# FASCICULE DE BREVET EUROPEEN

(11) **EP 1 552 813 B1**
(45) Date de publication et mention de la délivrance du brevet: **12.03.2008**
(21) Numéro de dépôt: 05290033.9
(22) Date de dépôt: 06.01.2005
(51) Int. Cl.: A61K 8/49, A61Q 5/06

(54) **Composition tinctoriale capillaire comprenant au moins un composé fluorindinium**
Haarfärbemittel enthaltend eine Fluorindinium-Verbindung
Hair dyeing composition comprising a fluorindinium compound.

(30) Priorité: 07.01.2004 FR 0450041
(43) Date de publication de la demande: 13.07.2005
(73) Titulaire: L'ORÉAL, 75008 Paris (FR)
(72) Inventeur: Lagrange, Alain, 77700 Coupvray (FR)
(74) Mandataire: Wattremez, Catherine

(56) Documents cités:
- WO-A-99/20233
- US-A- 3 390 948
- US-B1- 6 193 912

## Description

La présente invention a pour objet l'utilisation pour la teinture de fibres kératiniques, notamment humaines, comprenant à titre de colorant direct, d'au moins un composé de type fluorindine, ainsi qu'une composition tinctoriale le comprenant. Elle concerne de plus des composés en tant que tels.

Il existe essentiellement deux méthodes pour teindre les fibres kératiniques, notamment humaines, et plus particulièrement les cheveux.

La première, aussi appelée coloration permanente, consiste à appliquer sur les fibres un ou plusieurs précurseurs de colorants d'oxydation (ou bases d'oxydation) éventuellement associés à au moins un coupleur qui permet de faire varier la nuance obtenue avec le ou les précurseurs, en présence d'un agent oxydant. Ces deux types de composés sont des substances incolores ou faiblement colorées qui, associées à des produits oxydants, conduisent par un processus de condensation oxydative à des composés colorés.

La coloration permanente permet grâce à de multiples combinaisons entre les divers bases d'oxydation et coupleurs, d'obtenir une large palette de couleurs. Ces composés peuvent poser, dans certains cas, des problèmes d'innocuité. En outre, on peut aboutir à des colorations dont les ténacités sont difficiles à maîtriser, ce qui peut conduire à des virages sélectifs non souhaités de la couleur. Enfin, la présence d'un agent oxydant peut conduire à une altération de la fibre kératinique.

La deuxième méthode, appelée aussi coloration semi-permanente, consiste à mettre en oeuvre au moins un colorant direct, qui est un composé coloré et colorant les fibres kératiniques. De tels composés peuvent éventuellement être appliqués sur les fibres en présence d'un agent oxydant. Dans un tel cas, on parle de coloration directe éclaircissante.

L'un des principaux inconvénients de la coloration directe est son manque de ténacité, notamment vis-à-vis des shampooings.

Par ailleurs, ce type de coloration permet difficilement d'obtenir des nuances naturelles ou grises, de bonne qualité.

La présente invention a donc pour objet l'utilisation pour la teinture de fibres kératiniques, notamment humaines, d'une composition tinctoriale comprenant dans un milieu approprié pour la teinture, à titre de colorant direct, au moins un composé de formule (I) suivante :

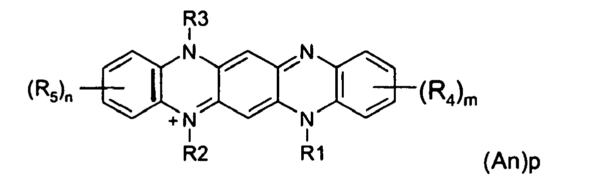

Formule dans laquelle :
R₁, R₂, R₃, identiques ou différents, représentent un radical alkyle en C₁-C₂₄, linéaire ou ramifié ; un radical aryle en C₆-C₃₀ ; un radical arylalkyle dans lequel la partie aryle est en C₆-C₃₀ et la partie alkyle, linéaire ou ramifiée est en C₁-C₂₄ ;
R₄, R₅, identiques ou non, représentent un atome d'hydrogène ; un atome d'halogène ; un radical hydroxy ; un radical alkyle en C₁-C₂₄, linéaire ou ramifié ;
un radical alcoxy en C₁-C₂₄, linéaire ou ramifié ; un groupement monohydroxy alcoxy dont la partie alkyle est en C₁-C₆, linéaire ou ramifiée ; un groupement polyhydroxy alcoxy, dans lequel la partie alkyle est en C₁-C₆, linéaire ou ramifiée ;
un groupement amino substitué ou non par un ou plusieurs radicaux alkyle en C₁-C₆, identiques ou non, linéaires ou ramifiés, substitués ou non par un ou plusieurs groupement hydroxyle ; un groupement thiol ; un groupement alkylthio dans lequel la partie alkyle est en C₁-C₆, linéaire ou ramifiée ; un groupement carboxylique sous forme acide ou salifiée (avec un métal alcalin ou un ammonium substitué ou non) ; un groupement alcoxycarbonyle dont la partie alkyle est en C₁-C₆, linéaire ou ramifiée ; un groupement alkylamide dont la partie alkyle est en C₁-C₆, linéaire ou ramifiée ; un groupement alkylcarbamyle dont la partie alkyle est en C₁-C₆, linéaire ou ramifiée ; un groupement cyano ; un groupement nitro ; un groupement sulfonyle ; un groupement alkylsulfonyle dont la partie alkyle est en C₁-C₆, linéaire ou ramifiée ; un groupement alkylsulfonylamido dont la partie alkyle est en C₁-C₆, linéaire ou ramifiée ; un radical aryle en C₆-C₃₀ éventuellement substitué par un ou plusieurs radicaux alkyle en C₁-C₆ ; un radical arylalkyle dont la partie aryle est en C₆-C₃₀, éventuellement substituée par un ou plusieurs radicaux alkyle en C₁-C₆ et la partie alkyle, linéaire ou ramifiée, est en C₁-C₂₄,
les radicaux alkyle ou aryle tels quels ou les parties alkyle des radicaux composés étant éventuellement substitués par un ou plusieurs groupements choisis parmi un groupement hydroxyle, un groupement -SO₃⁻ , un groupement -COO⁻, un atome d'halogène, un groupement alcoxy en C₁-C₆ ; un groupement monohydroxy alcoxy dont la partie alkyle en C₁-C₆, linéaire ou ramifiée ; un groupement polyhydroxy alcoxy, dans lequel la partie alkyle est en C₁-C₆, linéaire ou ramifiée ; un groupement amino substitué ou non par un ou plusieurs radicaux alkyles en C₁-C₆, identiques ou non, linéaires ou ramifiés, substitué ou non par un ou plusieurs groupement hydroxyle
n, m, indépendamment l'un de l'autre, représentent un nombre entier compris entre 0 et 4,
An représente un anion ou un mélange d'anions cosmétiquement acceptables ; p
valant 0 ou 1, afin de respecter l'électroneutralité du composé.

Elle a de plus pour objet une composition tinctoriale comprenant, dans un milieu approprié pour la teinture, au moins un composé de formule (I) précité et au moins un composé choisi parmi les polymères.

Elle a enfin pour objet un procédé de teinture des matières kératiniques, notamment humaines, mettant en oeuvre les composés de formule (I)

On a en effet constaté de manière surprenante que l'utilisation d'une telle composition tinctoriale permettait d'obtenir des colorations intenses, même sur des cheveux non sensibilisés.

On obtient en particulier des bleus performants.

Par ailleurs, les composés de formule (I), outre leur innocuité, permettent d'obtenir des reflets bleus, chromatiques ou sombres, très puissants, peu sélectifs et tenaces aux diverses actions que peuvent subir les cheveux, comme notamment les shampooings, les permanentes, l'action de la lumière, de la sueur.

Mais d'autres caractéristiques et avantages de la présente invention apparaîtront plus clairement à la lecture de la description qui va suivre.

Comme cela a été indiqué auparavant, l'invention concerne l'utilisation d'une composition tinctoriale comprenant dans un milieu approprié, à titre de colorant direct, au moins un composé de formule (I) suivante :

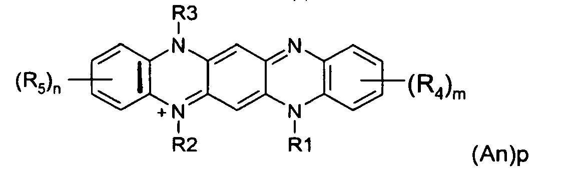

Formule dans laquelle :
R₁, R₂, R₃, identiques ou différents, représentent un radical alkyle en C₁-C₂₄, linéaire ou ramifié ; un radical aryle en C₆-C₃₀ ; un radical arylalkyle dans lequel la partie aryle est en C₆-C₃₀ et la partie alkyle, linéaire ou ramifiée est en C₁-C₂₄ ;
R₄, R₅, identiques ou non, représentent un atome d'hydrogène ; un atome d'halogène ; un radical hydroxy ; un radical alkyle en C₁-C₂₄, linéaire ou ramifié ;
un radical alcoxy en C₁-C₂₄, linéaire ou ramifié ; un groupement monohydroxy alcoxy dont la partie alkyle en C₁-C₆. linéaire ou ramifiée ; un groupement polyhydroxy alcoxy, dans lequel la partie alkyle est en C₁-C₆, linéaire ou ramifiée ;
un groupement amino substitué ou non par un ou plusieurs radicaux alkyle en C₁-C₆, identiques ou non, linéaires ou ramifiés, substitués ou non par un ou plusieurs groupement hydroxyle ; un groupement thiol ; un groupement alkylthio dans lequel la partie alkyle est en C₁-C₆, linéaire ou ramifiée ; un groupement carboxylique sous forme acide ou salifiée (avec un métal alcalin ou un ammonium substitué ou non) ; un groupement alcoxycarbonyle dont la partie alkyle est en C₁-C₆, linéaire ou ramifiée ; un groupement alkylamide dont la partie alkyle est en C₁-C₆, linéaire ou ramifiée ; un groupement alkylcarbamyle dont la partie alkyle est en C₁-C₆, linéaire ou ramifiée ; un groupement cyano ; un groupement nitro ; un groupement sulfonyle ; un groupement alkylsulfonyle dont la partie alkyle est en C₁-C₆, linéaire ou ramifiée ; un groupement alkylsulfonylamido dont la partie alkyle est en C₁-C₆, linéaire ou ramifiée ; un radical aryle en C₆-C₃₀ éventuellement substitué par un ou plusieurs radicaux alkyle en C₁-C₆ un radical arylalkyle dont la partie aryle est en C₆-C₃₀, éventuellement substituée par un ou plusieurs radicaux alkyle en C₁-C₆ et la partie alkyle, linéaire ou ramifiée, est en C₁-C₂₄,
les radicaux alkyle ou aryle tels quels ou les parties alkyle des radicaux composés étant éventuellement substitués par un ou plusieurs groupements choisis parmi un groupement hydroxyle, un groupement -SO₃⁻, un groupement -COO⁻, un atome d'halogène, un groupement alcoxy en C₁-C₆ ; un groupement monohydroxy alcoxy dont la partie alkyle en C₁-C₆, linéaire ou ramifiée ; un groupement polyhydroxy alcoxy, dans lequel la partie alkyle est en C₁-C₆, linéaire ou ramifiée ; un groupement amino substitué ou non par un ou plusieurs radicaux alkyles en C₁-C₆, identiques ou non, linéaires ou ramifiés, substitué ou non par un ou plusieurs groupement hydroxyle
n, m, indépendamment l'un de l'autre, représentent un nombre entier compris entre 0 et 4 ;
An représente un anion ou un mélange d'anions cosmétiquement acceptables ; p valant 0 ou 1, afin de respecter l'électroneutralité du composé.

Selon un mode de réalisation particulier de l'invention, dans la formule (I) les radicaux R₁, R₂, R₃, identiques ou différents, représentent un radical alkyle en C₁-C₆ ; un radical phényle ; un radical arylalkyle dans lequel la partie aryle est en C₆ et la partie alkyle est en C₁-C₆; les radicaux alkyle, phényle, arylalkyle étant éventuellement substitués par un ou plusieurs groupements choisis parmi les radicaux hydroxyle, -SO₃⁻, -COO⁻, halogène, alcoxy en C₁-C₆, amino, (di)alkylamino dont la partie alkyle est en C₁-C₆, (di)hydroxyalkylamino dont la partie alkyle est en C₁-C₆.

De préférence, les radicaux R₁, R₂, R₃, identiques ou différents, représentent les radicaux méthyle, éthyle, méthoxy, hydroxyéthyle, phényle, 3-sulfopropyle.

En ce qui concerne les radicaux R₄, R₅, ces derniers, identiques ou non, représentent, conformément à un mode de réalisation particulier de l'invention, un atome d'hydrogène ; un atome de chlore ; un radical amino ; un radical (di)alkylamino dont la partie alkyle est en C₁-C₆ ; un radical (di)hydroxyalkylamino dont la partie alkyle est en C₁-C₆ ; un radical hydroxy ; un radical alkyle en C₁-C₆, linéaire ou ramifié, un radical alcoxy en C₁-C₆, linéaire ou ramifié, lesdits radicaux alkyle ou alcoxy étant éventuellement substitué par un ou plusieurs groupements choisis parmi les radicaux hydroxy, halogène, alcoxy en C₁-C₆, amino, (di)alkylamino dont la partie alkyle est en C₁-C₆, (di)hydroxyalkylamino dont la partie alkyle est en C₁-C₆.

De préférence, les radicaux R₄, R₅, identiques ou non, représentent un atome d'hydrogène, un radical choisi parmi les groupements méthyle, éthyle, méthoxy, éthoxy, hydroxy, amino, diméthylamino, dihydroxyéthylamino, un atome de chlore.

An représente un anion ou un mélange d'anions, organiques ou minéraux permettant d'équilibrer la ou les charges des composés de formule (I) par exemple choisi parmi un halogénure tel que chlorure, bromure, fluorure, iodure ; un hydroxyde ; un sulfate ; un hydrogénosulfate ; un alkylsulfate pour lequel la partie alkyle, linéaire ou ramifiée, est en C₁-C₆, comme l'ion méthylsulfate ou éthylsulfate ; les carbonates et hydrogénocarbonates ; des sels d'acides carboxyliques tels que le formiate, l'acétate, le citrate, le tartrate, l'oxalate ; les alkylsulfonates pour lesquels la partie alkyle, linéaire ou ramifiée, est en C₁-C₆ comme l'ion méthylsulfonate ; les arylsulfonates pour lesquels la partie aryle, de préférence phényle, est éventuellement substituée par un ou plusieurs radicaux alkyle en C₁-C₄ tel que par exemple le 4-toluylsulfonate ; les alkylsulfonyles tel que le mésylate.

La teneur en composé de formule (I) présent dans la composition tinctoriale mise en oeuvre dans le procédé de l'invention, représente de préférence de 0,001 à 20 % en poids de ladite composition tinctoriale, de manière plus avantageuse de 0,01 à 10 % en poids par rapport au poids de la composition tinctoriale.

Ces composés de formule (I) sont connus en eux-mêmes. On peut notamment citer ceux décrits dans le brevet US 3390948.

Un autre objet de l'invention est constitué par des composés de formule (I) particulière. Plus spécialement, les composés correspondent à la formule (I') suivante :

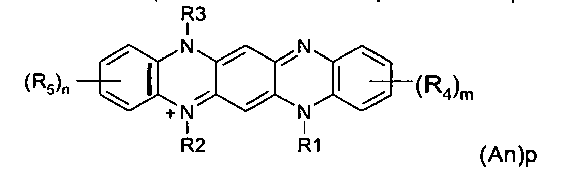

Formule dans laquelle :
- R₁, R₂, R₃, identiques ou différents, représentent un radical alkyle en C₁-C₂₄, linéaire
ou ramifié ; un radical aryle en C₆-C₃₀ ; un radical arylalkyle dans lequel la partie aryle est en C₆-C₃₀ et la partie alkyle, linéaire ou ramifiée est en C₁-C₂₄ ; à la condition que les radicaux R₁, R₃, identiques ou différents, représentent des radicaux alkyle porteurs d'au moins un groupement hydroxyle ;
- R₄, R₅, identiques ou non, représentent un atome d'hydrogène ; un atome d'halogène ; un radical hydroxyle ; un radical alkyle en C₁-C₂₄, linéaire ou ramifié ; un radical alcoxy en C₁-C₂₄, linéaire ou ramifié ; un groupement monohydroxy alcoxy dont la partie alkyle est en C₁-C₆, linéaire ou ramifiée ; un groupement polyhydroxy alcoxy, dans lequel la partie alkyle est en C₁-C₆, linéaire ou ramifiée ; un groupement amino substitué ou non par un ou plusieurs radicaux alkyle en C₁-C₆, identiques ou non, linéaires ou ramifiés, substitués ou non par un ou plusieurs groupement hydroxyle ; un groupement thiol ; un groupement alkylthio dans lequel la partie alkyle est en C₁-C₆, linéaire ou ramifiée ; un groupement carboxylique sous forme acide ou salifiée (avec un métal alcalin ou un ammonium substitué ou non); un groupement alcoxycarbonyle dont la partie alkyle est en C₁-C₆, linéaire ou ramifiée ; un groupement alkylamide dont la partie alkyle est en C₁-C₆, linéaire ou ramifiée ; un groupement alkylcarbamyle dont la partie alkyle est en C₁-C₆, linéaire ou ramifiée ; un groupement cyano ; un groupement nitro ; un groupement sulfonyle ; un groupement alkylsulfonyle dont la partie alkyle est en C₁-C₆, linéaire ou ramifiée ; un groupement alkylsulfonylamido dont la partie alkyle est en C₁-C₆, linéaire ou ramifiée ; un radical aryle en C₆-C₃₀ éventuellement substitué par un ou plusieurs radicaux alkyle en C₁-C₆ ; un radical arylalkyle dont la partie aryle est en C₆-C₃₀, éventuellement substituée par un ou plusieurs radicaux alkyle en C₁-C₆ et la partie alkyle, linéaire ou ramifiée, est en C₁-C24,
- les radicaux alkyle ou aryle tels quels ou les parties alkyle des radicaux composés étant éventuellement substitués par un ou plusieurs groupements choisis parmi un groupement hydroxyle, un groupement -SO₃⁻, -COO⁻, un atome d'halogène, un groupement alcoxy en C₁-C₆; un groupement monohydroxy alcoxy dont la partie alkyle en C₁-C₆, linéaire ou ramifiée ; un groupement polyhydroxy alcoxy, dans lequel la partie alkyle est en C₁-C₆, linéaire ou ramifiée ; un groupement amino substitué ou non par un ou plusieurs radicaux alkyles en C₁-C₆, identiques ou non, linéaires ou ramifiés, substitué ou non par un ou plusieurs groupement hydroxyle,
- n, m, indépendamment l'un de l'autre, représentent un nombre entier compris entre 0 et 4 ;
- An représente un anion ou un mélange d'anions cosmétiquement acceptables ; p valant 0 ou 1, afin de respecter l'électroneutralité du composé.

Les radicaux R₁ à R₄, n, m, An étant définis comme précédemment.

De préférence, ces composés correspondent à l'une des formules (i) (ii) (iii) suivantes :

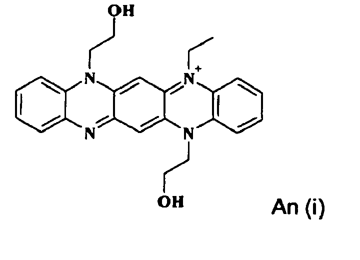

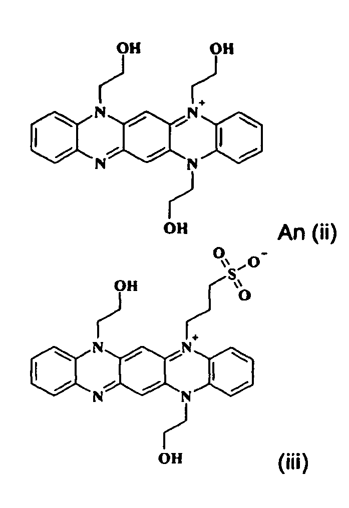

Ces composés sont obtenus de manière classique par l'homme du métier.

On pourra notamment se référer au brevet US 3390948 précité.

On peut par exemple utiliser comme réactif un composé de formule suivante :

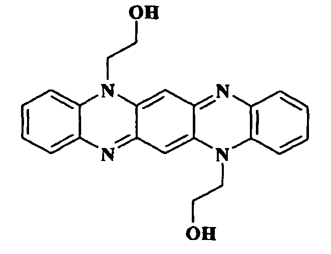

Puis effectuer sur ce composé une étape classique de quaternisation.

Un autre objet de la présente invention est constitué par une composition tinctoriale comprenant, à titre de colorant direct, au moins un composé de formule (I), et au moins un composé choisi parmi les polymères.

La composition comprend au moins un polymère, qui est choisi de préférence parmi les polymères épaississants associatifs ou non, parmi les polymères conditionneurs ou parmi les polymères fixants.

Il est précisé que les polymères associatifs sont des polymères hydrophiles capables, dans un milieu aqueux, de s'associer réversiblement entre eux ou avec d'autres molécules.

Leur structure chimique comprend plus particulièrement au moins une zone hydrophile et au moins une zone hydrophobe.

Les polymères associatifs présents dans la composition selon l'invention peuvent être de nature non ionique, anionique, cationique ou amphotère.

Parmi les dérivés de polyuréthanes associatifs, on peut citer les copolymères anioniques obtenus par polymérisation :
- environ 20% à 70% en poids d'un acide carboxylique à insaturation α,β-monoéthylénique,
- environ 20 à 80% en poids d'un monomère à insaturation α,β-monoéthylénique non-tensioactif différent du précédent,
- environ 0,5 à 60% en poids d'un mono-uréthane non-ionique qui est le produit de réaction d'un tensioactif monohydroxylé avec un monoisocyanate à insaturation monoéthylénique.

De tels sont notamment décrits dans EP 173109 et plus particulièrement dans l'exemple 3. Plus précisément, ce polymère est un terpolymère acide méthacrylique / acrylate de méthyle / diméthyl métaisopropényl benzyl isocyanate d'alcool béhényle éthoxylé (40OE) en dispersion aqueuse à 25%.Ce produit est proposé sous la référence VISCOPHOBE DB1000 par la Société AMERCHOL.

Conviennent aussi citer les polyuréthanes associatifs cationiques dont la famille a été décrite dans la demande FR 0009609.

Les dérivés de polyuréthanes associatifs de l'invention peuvent être aussi des polyuréthanes polyéthers non ioniques. Plus particulièrement, lesdits polymères comportent dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, ces polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non ioniques à chaîne grasse peuvent être des copolymères triblocs dont la séquence hydrophile est une chaîne polyoxyéthylénée comportant de 50 à 1000 groupements oxyéthylénés.

A titre d'exemples de polyéthers polyuréthanes non ioniques à chaîne hydrophobe utilisables dans l'invention, on peut utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂-C₁₄ et le produit ELFACOS T212® à chaîne alkyle en C₁₈ de chez AKZO.
Le produit DW 1206B® de chez ROHM & HAAS à chaîne alkyle en C₂₀ et à liaison uréthane, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemples, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention peuvent aussi être choisis parmi ceux décrits dans l'article de G. Fonnum, J. Bakke et Fk. Hansen - Colloid Polym. Sci 271, 380-389 (1993).

Plus particulièrement encore, on peut utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Parmi les polymères dérivés de celluloses associatifs utilisables selon l'invention on peut citer :
- les celluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci,
- les hydroxyéthylcelluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci.
   Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
   On peut indiquer comme exemples d'alkylhydroxyéthylcelluloses quaternisées à chaîne hydrophobe en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C18) commercialisés par la société CRODA.
- les dérivés de cellulose non ioniques tels que les hydroxyéthylcelluloses modifiées par des groupements comportant au moins une chaîne hydrophobe tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
- les dérivés de cellulose modifiée par des groupes polyalkylène glycol éther d'alkyl phénol, tel que le produit AMERCELL POLYMER HM-1500® vendu par la société AMERCHOL.

En ce qui concerne les polyvinyllactames associatifs utilisables dans le cadre de la présente invention, ces derniers peuvent notamment être choisis parmi ceux décrits dans FR 0101106.

On peut aussi citer les terpolymères comprenant, en poids, 40 à 95% de monomère (a), 0,1 à 55% de monomère (c) et 0,25 à 50% de monomère (b).

De tels polymères sont notamment décrits dans la demande de brevet WO 00/68282 dont le contenu fait partie intégrante de l'invention.

Comme polymères poly(vinyllactame) selon l'invention, on utilise notamment les terpolymères vinylpyrrolidone / diméthylaminopropylméthacrylamide / tosylate de dodécyl diméthylméthacrylamidopropylammonium, les terpolymères vinylpyrrolidone / diméthyl aminopropylméthacrylamide / tosylate de cocoyldiméthylméthacrylamido propyl ammonium, les terpolymères vinylpyrrolidone / diméthylaminopropyl méthacrylamide / tosylate ou chlorure de lauryldiméthylméthacrylamidopropylammonium.

Les dérivés de polyvinyllactames associatifs de l'invention peuvent être aussi des copolymères non ioniques de vinylpyrrolidone et de monomères hydrophobes à chaîne hydrophobe dont on peut citer à titre d'exemple :
- les produits ANTARON V216® ou GANEX V216® (copolymère vinylpyrrolidone / hexadécène) vendu par la société I.S.P.
- les produits ANTARON V220® ou GANEX V220® (copolymère vinylpyrrolidone / eicosène) vendu par la société I.S.P.

Parmi les dérivés de polyacides insaturés associatifs on peut citer ceux comportant au moins un motif hydrophile de type acide carboxylique insaturé oléfinique, de préférence les acides acrylique, méthacrylique, éthacrylique, et au moins un motif hydrophobe de type ester d'alkyl (C₁₀-C₃₀) d'acide carboxylique insaturé.

Des polymères anioniques de ce type sont par exemple décrits et préparés, selon les brevets US-3 915 921 et 4 509 949.

Dans ce type de polymères associatifs anioniques, on utilise plus particulièrement des polymères formés à partir d'un mélange de monomères comprenant :
(i) essentiellement de l'acide acrylique,
(ii) un (méth)acrylate d'alkyle ayant 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on préfère ceux constitués de 95 à 60% en poids d'acide acrylique (motif hydrophile), de 4 à 40% en poids d'acrylate d'alkyles en C₁₀-C₃₀ (motif hydrophobe), et de 0 à 6% en poids de monomère polymérisable réticulant, ou bien ceux constitués de 98 à 96% en poids d'acide acrylique (motif hydrophile), de 1 à 4% en poids d'acrylate d'alkyles en C₁₀-C₃₀ (motif hydrophobe), et de 0,1 à 0,6% en poids de monomère polymérisable réticulant tel que ceux décrits précédemment.

Parmi lesdits polymères ci-dessus, on préfère tout particulièrement les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.

Parmi les dérivés de polyacides insaturés associatifs on peut aussi citer ceux comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemples de ce type de composés, on peut citer l'ACULYN 22® vendu par la société ROHM et HAAS, qui est un terpolymère acide méthacrylique/acrylate d'éthyle/méthacrylate de stéaryle oxyalkyléné.

En ce qui concerne les polymères épaississants non associatifs, ces derniers ne contiennent pas de chaîne grasse en C₁₀-C₃₀.

Parmi les polymères épaississants non associatifs, on peut citer les homopolymères d'acide acrylique réticulés, par exemple par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA.

On peut aussi citer, parmi les polymères épaississants non associatifs, les homopolymères réticulés d'acide 2-acrylamido-2-méthyl-propane sulfonique et leurs copolymères réticulés d'acrylamide partiellement ou totalement neutralisés. Conviennent notamment, les homopolymères décrits dans la demande EP 815 828 à laquelle on pourra se reporter à ce sujet. Parmi les copolymères réticulés d'acide 2-acrylamido-2-méthyl-propane sulfonique et d'acrylamide partiellement ou totalement neutralisés, on peut citer en particulier le produit décrit dans l'exemple 1 du document EP 503 853 et l'on pourra se reporter à ce document pour ce qui a trait à ces polymères. Il est à noter que dans le cas où les composés sont neutralisés, ils le sont notamment par l'emploi d'une base telle que l'hydroxyde de sodium ou de potassium ou une amine.

Conviennent aussi, en tant que polymères épaississants non associatifs, les homopolymères d'acrylate d'ammonium ou les copolymères d'acrylate d'ammonium et d'acrylamide.

A titre d'exemples d'homopolymères d'acrylate d'ammonium, on peut citer le produit vendu sous le nom MICROSAP PAS 5193 par la société HOECHST. Parmi les copolymères d'acrylate d'ammonium et d'acrylamide, on peut citer le produit vendu sous le nom BOZEPOL C NOUVEAU ou le produit PAS 5193 vendus par la société HOECHST. On pourra notamment se référer aux documents FR 2 416 723, US 2798053 et US 2923692 pour ce qui a trait à la description et à la préparation de tels composés.

Les homopolymères de diméthylaminoéthyl-méthacrylate quaternisé par le chlorure de méthyle ou les copolymères de diméthylamino-éthylméthacrylate quaternisé par le chlorure de méthyle et d'acrylamide peuvent aussi être utilisés comme polymères épaississants non associatifs.

Parmi les homopolymères de cette famille, on peut citer les produits vendus sous les noms SALCARE 95 et SALCARE 96 par la société CIBA-ALLIED COLLOIDS. Parmi les copolymères de cette famille, on peut citer le produit SALCARE SC92 vendu par CIBA-ALLIED COLLOIDS ou le produit PAS 5194 vendu par HOECHST. Ces polymères sont notamment décrits et préparés dans le document EP 395282 auquel on pourra se référer.

Conviennent également les gommes de guar non ioniques non modifiées telles que celles vendues sous la dénomination VIDOGUM GH 175 par la société UNIPECTINE et sous la dénomination JAGUAR C par la société MEYHALL.

Il est de même possible d'utiliser des gommes de guar non ioniques modifiées par des groupements hydroxyalkyle en C₁-C₆ (notamment hydroxyméthyle, hydroxyéthyle, hydroxypropyle et hydroxybutyle).

De telles gommes de guar non ioniques modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL ou sous la dénomination GALACTASOL 4H4FD2 par la société AQUALON.

Conviennent aussi les gommes de biopolysaccharides d'origine microbienne, comme par exemple les gommes de scléroglucane ou de xanthane ; les gommes issues d'exudats végétaux telles que les gommes arabiques, gommes Ghatti, gommes Karaya et Tragacanthe ; les hydroxypropyl ou carboxyméthyl celluloses ; les pectines et les alginates. De tels composés sont notamment décrits dans l'ouvrage de Robert L. DAVIDSON intitulé "Handbook of Water soluble gums and resins" édité chez Mc Graw Hill Book Company (1980).

La concentration en polymère(s) épaississant(s) associatif(s) ou non, présent(s) dans la composition tinctoriale peut varier de 0,01 à 10% en poids, plus particulièrement de 0,1 à 5% en poids, par rapport au poids de la composition tinctoriale.

Comme indiqué auparavant, la composition selon l'invention peut comprendre au moins un polymère conditionneur ou un polymère fixant.

Il est tout d'abord rappelé que l'on entend par agent conditionneur tout agent ayant pour fonction l'amélioration des propriétés cosmétiques des matières kératiniques telles que les cheveux, en particulier la douceur, le démêlage, le toucher, le lissage, l'électricité statique.

Les polymères conditionneurs convenables sont choisis parmi les polymères cationiques et les polyorganosiloxanes cationiques ou non ioniques.

Par polymère cationique, on entend tout polymère contenant des groupes cationiques et/ou des groupes ionisables en groupes cationiques.

Ces polymères cationiques sont choisis parmi tous ceux déjà connus en tant que tels pour leur capacité d'améliorer les propriétés cosmétiques des cheveux traités par des compositions détergentes. On peut citer notamment ceux décrits dans les demandes de brevet EP 0 337 354, FR 2 270 846, FR 2 383 660, FR 2 598 611, FR 2 470 596 et FR 2519863.

Les polymères cationiques préférés sont choisis parmi ceux qui contiennent des motifs comportant des groupements amines primaires, secondaires, tertiaires et/ou quaternaires qui font partie de la chaîne macromoléculaire principale, ou bien sont portés par des groupes latéraux directement reliés à celle-ci.

Parmi les polymères cationiques, on peut citer plus particulièrement les polymères du type polyamine, polyaminoamide et polyammonium quaternaire. Il s'agit de produits connus.

Les polymères du type polyamine, polyaminoamide et polyammonium quaternaire, que l'on peut utiliser dans les compositions de la présente invention, sont ceux décrits dans les brevets FR 2 505 348 et FR 2 542 997. Parmi ces polymères, on peut citer en particulier :
**(1)** les homopolymères ou copolymères dérivés d'esters ou d'amides acryliques ou méthacryliques et comportant au moins un des motifs de formules suivantes: 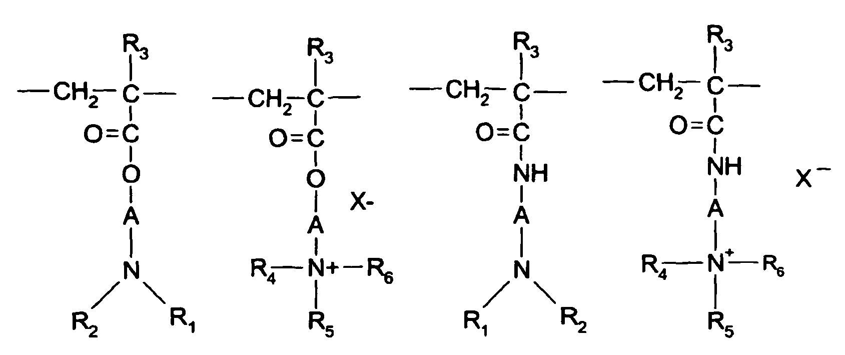 dans lesquelles:
   R₃, identiques ou différents, désignent un atome d'hydrogène ou un groupe CH₃ ;
   A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone;
   R₁ et R₂, identiques ou différents, représentent un atome d'hydrogène ou un groupe alkyle ayant de 1 à 6 atomes de carbone, et de préférence un groupe méthyle ou éthyle;
   X- désigne un anion dérivé d'un acide minéral ou organique tel qu'un anion méthosulfate ou un halogénure comme le chlorure ou le bromure.

   Les copolymères de la famille **(1)** peuvent contenir en outre un ou plusieurs motifs dérivant de comonomères choisis dans la famille des acrylamides, méthacrylamides, diacétone-acrylamides, acrylamides et méthacrylamides substitués sur l'atome d'azote par des groupes alkyle inférieur en C₁-C₄, des groupes dérivés des acides acryliques ou méthacryliques ou de leurs esters, de vinyllactames tels que la vinylpyrrolidone ou le vinylcaprolactame, d'esters vinyliques.
   Parmi ces copolymères de la famille **(1)**, on peut citer en particulier :
   - les copolymères d'acrylamide et de méthacrylate de diméthylaminoéthyle quaternisé au sulfate de diméthyle ou avec un halogénure de méthyle, tels que celui vendu sous la dénomination HERCOFLOC^{®} par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINAQUAT^{®} P 100 par la société CIBA GEIGY,
   - le copolymère d'acrylamide et de méthosulfate de méthacryloyloxyéthyltriméthylammonium vendu sous la dénomination RETEN^{®} par la société HERCULES,
   - les copolymères vinylpyrrolidone/acrylate ou méthacrylate de dialkylaminoalkyle quaternisés ou non, tels que les produits vendus sous la dénomination GAFQUAT^{®} par la société ISP comme, par exemple, GAFQUAT^{®} 734 ou GAFQUAT^{®} 755, ou bien les produits dénommés COPOLYMER 845, 958 et 937. Ces polymères sont décrits en détail dans les brevets FR 2 077 143 et FR 2 393 573,
   - les terpolymères méthacrylate de diméthylaminoéthyle/ vinylcaprolactame /vinylpyrrolidone tels que le produit vendu sous la dénomination GAFFIX^{®} VC 713 par la société ISP,
   - les copolymères vinylpyrrolidone/méthacrylamidopropyl-di-méthylamine commercialisés notamment sous la dénomination STYLEZE^{®} CC 10 par ISP, et
   - les copolymères vinylpyrrolidone/méthacrylamide de diméthylaminopropyle quaternisé, tels que le produit vendu sous la dénomination GAFQUAT^{®} HS 100 par la société ISP.
**(2)** Les dérivés d'éthers de cellulose comportant des groupements ammonium quaternaires décrits dans le brevet FR 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
**(3)** Les dérivés cationiques de la cellulose tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyltriméthylammonium, de méthacrylamidopropyl-triméthylammonium, de diméthyldiallylammonium.
   Les produits commercialisés répondant à cette définition sont plus particulièrement les produits vendus sous la dénomination Celquat^{®} L 200 et Celquat^{®} H 100 par la Société National Starch.
**(4)** Les polysaccharides cationiques décrits plus particulièrement dans les brevets US 3 589 578 et US 4 031 307, tels que les gommes de guar contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR^{®} C13 S, JAGUAR^{®} C 15, JAGUAR^{®} C 17 ou JAGUAR^{®} C162 par la société MEYHALL.
**(5)** Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets FR 2 162 025 et FR 2 280 361.
**(6)** Les polyaminoamides solubles dans l'eau, préparés en particulier par polycondensation d'un composé acide avec une polyamine. Ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé, l'agent réticulant étant utilisé dans des proportions allant de 0,025 à 0,35 mole par groupement amine du polyaminoamide. Ces polyaminoamides peuvent être alkylés ou, s'ils comportent une ou plusieurs fonctions amines tertiaires, quaternisées. De tels polymères sont notamment décrits dans les brevets FR 2 252 840 et FR 2 368 508.
**(7)** Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle ou propyle, et le groupe alkylène comporte de 1 à 4 atomes de carbone, et désigne de préférence le groupe éthylène. De tels polymères sont notamment décrits dans le brevet FR 1 583 363.
   Parmi ces dérivés, on peut citer plus particulièrement les polymères acide adipique/diméthylaminohydroxypropyl-diéthylène-triamine vendus sous la dénomination Cartaretine^{®} F, F4 ou F8 par la société Sandoz.
**(8)** Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone, le rapport molaire de la polyalkylène-polylamine à l'acide dicarboxylique étant compris entre 0,8:1 et 1,4:1. Le polyaminoamide résultant de cette réaction est ensuite amené à réagir avec l'épichlorhydrine dans un rapport molaire d'épichlorhydrine par rapport au groupement amine secondaire du polyaminoamide compris entre 0,5:1 et 1,8:1. De tels polymères sont notamment décrits dans les brevets US 3 227 615 et US 2 961 347.
   Des polymères de ce type sont en particulier commercialisés sous la dénomination Hercosett^{®} 57 par la société Hercules Inc. ou bien sous la dénomination de PD 170 ou Delsette^{®} 101 par la société Hercules dans le cas du copolymère d'acide adipique/époxypropyl-diéthylène-triamine.
**(9)** Les cyclopolymères d'alkyldiallylamine ou de dialkyldiallylammonium tels que les homopolymères ou copolymères comportant, comme constituant principal de la chaîne, des motifs répondant aux formules (Va) ou (Vb) : 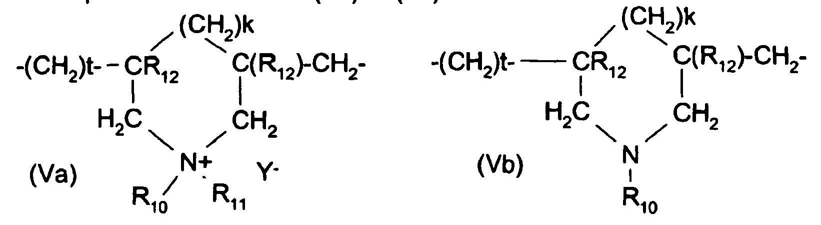 dans lesquelles
   k et t sont égaux à 0 ou 1, la somme k + t étant égale à 1 ;
   R₁₂ désigne un atome d'hydrogène ou un groupe méthyle ;
   R₁₀ et R₁₁, indépendamment l'un de l'autre, désignent un groupement alkyle ayant de 1 à 22 atomes de carbone, un groupement hydroxyalkyle en C₁₋₅, un groupement amidoalkyle inférieur en C₁-C₄, ou bien R₁₀ et R₁₁ peuvent désigner conjointement avec l'atome d'azote auquel ils sont rattachés, des groupements hétérocycliques, tels que pipéridinyle ou morpholinyle ;
   Y⁻ est un anion tel que bromure, chlorure, acétate, borate, citrate, tartrate, bisulfate, bisulfite, sulfate, phosphate.

   Ces polymères sont notamment décrits dans le brevet FR 2 080 759 et dans son certificat d'addition 2 190 406.
   Parmi les polymères définis ci-dessus, on peut citer plus particulièrement l'homopolymère de chlorure de diméthyldiallyl-ammonium vendu sous la dénomination MERQUAT^{®} 100 par la société CALGON (et ses homologues de faibles masses moléculaires moyenne en poids) et les copolymères de chlorure de diallyldiméthylammonium et d'acrylamide commercialisés sous la dénomination MERQUAT^{®} 550.
**(10)** Les polymères de diammonium quaternaire contenant des motifs récurrents répondant à la formule (VI): 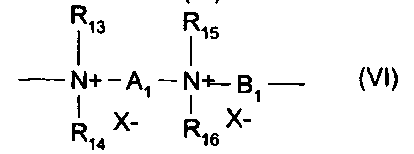 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkyle aliphatiques inférieurs, ou bien R₁₃, R₁₄, R₁₅ et R₁₆, ensemble ou séparément, forment avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁₋₆, linéaire ou ramifié, substitué par un groupement nitrile, ester, acyle, amide ou -CO-O-R₁₇-D ou -CO-NH-R₁₇-D où R₁₇ est un groupe alkylène et D un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et X- désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-D-OC-(CH₂)ₙ-

      dans lequel D désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
      d) un groupement uréylène de formule -NH-CO-NH- .

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets FR 2 320 330, FR 2 270 846, FR 2 316 271, FR 2 336 434 et FR 2 413 907 et les brevets US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945 et US 4 027 020.
   On peut utiliser plus particulièrement les polymères qui sont constitués de motifs récurrents répondant à la formule : 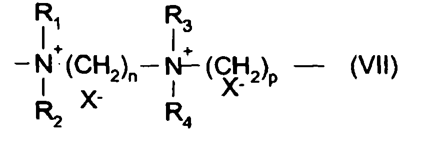 dans laquelle R₁, R₂, R₃ et R₄, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (VII) particulièrement préféré est celui pour lequel R₁, R₂, R₃ et R₄ représentent un groupe méthyle et n=3, p=6 et X=Cl, dénommé chlorure d'hexadiméthrine (CTFA).
**(11)** Les polymères de polyammonium quaternaire, constitués de motifs de formule (VIII) : 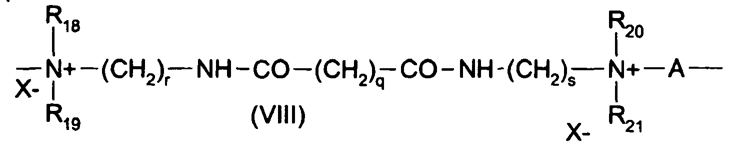 dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogénure,
   A désigne un radical d'un dihalogénure ou représente de préférence -CH₂-CH₂-O-CH₂-CH₂-.

   De tels composés sont notamment décrits dans la demande de brevet EP-A-122 324.
   On peut citer parmi ceux-ci, par exemple, les produits Mirapol® A 15, Mirapol® AD1, Mirapol® AZ1 et Mirapol® 175 vendus par la société Miranol.
**(12)** Les polymères quaternaires de vinylpyrrolidone et de vinylimidazole tels que, par exemple, les produits commercialisés sous les dénominations Luviquat^{®} FC 905, FC 550 et FC 370 par la société B.A.S.F. On peut citer notamment les copolymères de vinylpyrrolidone et de chlorure de méthylvinylimidazolium.
**(13)** Les polyamines comme le Polyquart^{®} H vendu par HENKEL, référencées sous le nom de POLYETHYLENEGLYCOL (15) TALLOW POLYAMINE dans le dictionnaire CTFA.
**(14)** Les polymères réticulés ou non réticulés de sels de méthacryloyloxyalkyl(C₁₋₄) trialkyl(C₁₋₄)ammonium, tels que les polymères obtenus par homopolymérisation du méthacrylate de diméthylaminoéthyle quaternisé par le chlorure de méthyle, ou par copolymérisation de l'acrylamide et de méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide. On peut plus particulièrement utiliser un copolymère réticulé acrylamide/chlorure de méthacryloyloxy-éthyl-triméthylammonium (20/80 en poids) sous forme de dispersion contenant 50 % en poids dudit copolymère dans de l'huile minérale. Cette dispersion est commercialisée sous le nom de SALCARE^{®} SC 92 par la Société ALLIED COLLOIDS. On peut également utiliser un homopolymère réticulé du chlorure de méthacryloyloxyéthyl-triméthylammonium contenant environ 50 % en poids de l'homopolymère dans de l'huile minérale ou dans un ester liquide. Ces dispersions sont commercialisées sous les noms de SALCARE^{®} SC 95 et SALCARE^{®} SC 96 par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés cationiques de la chitine.

En ce qui concerne les polyorganosiloxanes (ou organosiloxanes ou silicones) aminés ou non, de préférence non volatiles, ils peuvent aussi être utilisés comme agent conditionneur. Ils peuvent se présenter en particulier sous forme d'huiles, de cires, de résines ou de gommes.

On peut citer tout particulièrement les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles Silbione^{®} des séries 47 et 70 047 ou les huiles Mirasil^{®} commercialisées par Rhodia Chimie telles que par exemple l'huile 70 047 V 500 000 ;
- les huiles de la série Mirasil commercialisées par la société Rhodia Chimie ;
- les huiles de la série 200 de la société Dow Corning telles que plus particulièrement la DC200 de viscosité 60 000 cSt (mm²/s);
- les huiles Viscasil^{®} de General Electric et certaines huiles des séries SF (SF 96, SF 18) de General Electric.

On peut également citer les polydiméthylsiloxanes à groupements terminaux diméthylsilanol (Dimethiconol selon la dénomination CTFA) tels que les huiles de la série 48 de la société Rhodia Chimie ; mais aussi les produits commercialisés sous les dénominations "Abil^{®} Wax 9800 et 9801" par la société Goldschmidt qui sont des polyalkyl (C₁-C₂₀) siloxanes.

Les polyalkylarylsiloxanes peuvent être plus particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles Silbione de la série 70 641 de Rhodia Chimie ;
- les huiles des séries Rhodorsil 70 633 et 763 de Rhodia Chimie ;
- l'huile Dow Corning 556 Cosmetic Grad Fluid de Dow Corning ;
- les silicones de la série PK de Bayer comme le produit PK20 ;
- les silicones des séries PN, PH de Bayer comme les produits PN1000 et PH1000 ;
- certaines huiles des séries SF de General Electric telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants de type polydiméthylsiloxane, les gommes polydiméthylsiloxane / méthylvinylsiloxane, les polydiméthylsiloxane / diphénylsiloxane, les polydiméthylsiloxane / phénylméthylsiloxane, les polydiméthylsiloxane / diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société Dow Corning ;
- les mélanges formés à partir d'une gomme polydiméthylsiloxane avec une silicone cyclique tel que le produit SF 1214 Silicone Fluid de la société General Electric, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société General Electric. Le produit SF 1236 est le mélange d'une gomme SE 30 définie ci-dessus ayant une viscosité de 20 m²/s et d'une huile SF 96 d'une viscosité de 5.10-⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "Dow Corning 593" ou ceux commercialisés sous les dénominations "Silicone Fluid SS 4230 et SS 4267" par la société General Electric et qui sont des silicones de structure diméthyl/triméthyl siloxane.

On peut également citer les résines du type triméthylsiloxysilicate commercialisées notamment sous les dénominations X22-4914, X21-5034 et X21-5037 par la société Shin-Etsu.

Les silicones organo-modifiées utilisables conformément à l'invention sont des silicones telles que définies précédemment et comportant dans leur structure un ou plusieurs groupements organofonctionnels fixés par l'intermédiaire d'un radical hydrocarboné. Ces groupements organofonctionnels sont différents des groupements aminés.

Parmi les silicones organo-modifiées différentes de celles de formules (I) ou (II), on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société Dow Corning sous la dénomination DC 1248 et l'alkyl (C₁₂) méthicone copolyol commercialisée par la société Dow Corning sous la dénomination Q2 5200 ;
- des groupements thiols, comme les produits commercialisés sous les dénominations "GP 72 A" et "GP 71" de Genesee ;
- des groupements alcoxylés, comme le produit commercialisé sous la dénomination "Silicone Copolymer F-755" par SWS Silicones et Abil Wax 2428, 2434 et 2440 par la société Goldschmidt ;
- des groupements hydroxylés, comme les polyorganosiloxanes à fonction hydroxyalkyle décrits dans FR 8516334 ;
- des groupements acyloxyalkyle, tels que par exemple les polyorganosiloxanes décrits dans US 4957732 ;
- des groupements anioniques du type carboxylique, comme par exemple dans les produits décrits dans EP 186507 de la société Chisso Corporation, ou de type alkylcarboxyliques comme ceux présents dans le produit X-22-3701 E de la société Shin-Etsu ; 2-hydroxyalkylsulfonate ; 2-hydroxyalkylthiosulfate tels que les produits commercialisés par la société Goldschmidt sous les dénominations "Abil S201" et "Abil S255".

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans EP 412704, EP 412707, EP 640105 et WO 95/00578, EP 582152, WO 93/23009, US 4693935, US 4728571 et US 4972037. Ces polymères sont de préférence anioniques ou non ioniques.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Il est à noter que les silicones peuvent également être utilisées sous forme d'émulsions, de nanoémulsions ou de micrémulsions.

Par polymères fixants, on entend au sens de la présente invention tout polymère permettant de donner ou de maintenir une forme à la chevelure.

De préférence, les polymères fixants susceptibles d'être utilisés sont choisis parmi les polymères anioniques, amphotères, non ioniques et leurs mélanges.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 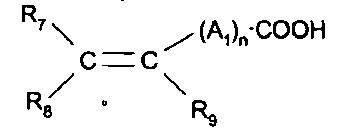 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID et ULTRAHOLD^{®} par la société BASF, les copolymères d'acide acrylique et d'acrylamide, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Un produit commercial entrant dans cette classe est la résine 28-29-30 commercialisée par la société National Starch.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ^{®} AN ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
      les fonctions anhydrides de ces copolymères étant éventuellement monoestérifiées
   ou monoamidifiées.
   Ces polymères sont par exemple décrits dans les brevets français n^{os} 2 350 384 et 2 357 241 de la demanderesse.
E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous la dénomination Flexan^{®} 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique.

Selon l'invention, parmi les polymères fixants anioniques cités ci-dessus, on préférera les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique et les copolymères acétate de vinyle/acide crotonique greffés par du polyéthylèneglycol vendus sous la dénomination ARISTOFLEX^{®} A par la société BASF.

Les polymères fixants anioniques cités ci-dessus, les plus particulièrement préférés sont choisis parmi les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF, les terpolymères vinylpyrrolidone/acide acrylique/méthacrylate de lauryle vendus sous la dénomination ACRYLIDONE^{®} LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
(3) les polyaminoamides réticulés et acylés partiellement ou totalement dérivant de poyaminoamides de formule générale : 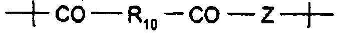 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 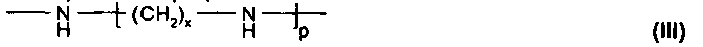
      où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (III) ci-dessus, dans lequel x=2 et p=1 et qui dérive de l'éthylène-diamine, ou le groupe dérivant de la pipérazine : 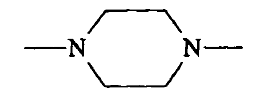
   c) dans les proportions de 0 à 20 % en moles, le groupe -NH-(CH₂)₆-NH- dérivant de l'hexaméthylènediamine,
   ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 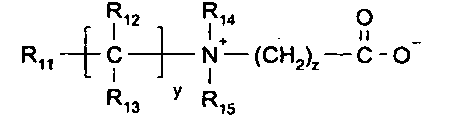 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonioéthyl méthacrylate de méthyle.
(5) les polymères dérivés du chitosane comportant des motifs monomères répondant aux formules suivantes : 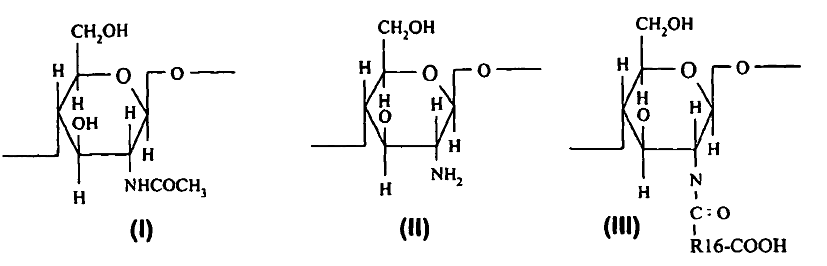 le motif (I) étant présent dans des proportions comprises entre 0 et 30%, le motif (II) dans des proportions comprises entre 5 et 50% et le motif (III) dans des proportions comprises entre 30 et 90%, étant entendu que dans ce motif (III), R₁₆ représente un groupe de formule : 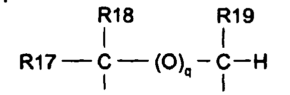 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 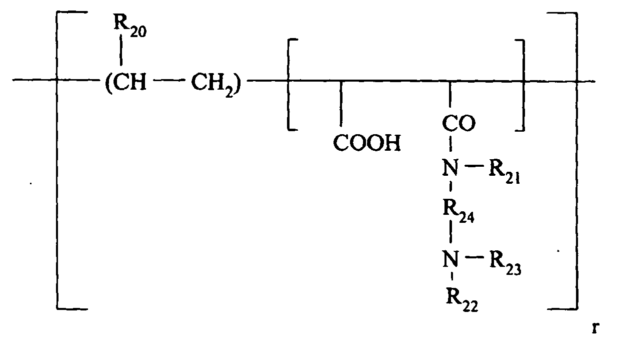 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ repré-sentant un groupement -CH₂-CH₂-, -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane.
(8) Les polymères amphotères du type -D-X-D-X choisis parmi:
   a) les polymères obtenus par action de l'acide chloracétique ou le chloracétate de sodium sur les composés comportant au moins un motif de formule:

      -D-X-D-X-D- (VI)

      où D désigne un groupe 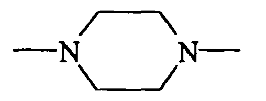 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')

      où D désigne un groupe 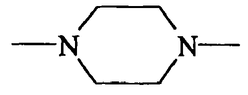 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthyl-carboxyméthyl-ammonio-éthylméthacrylate de méthyle.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque, par exemple, de maléate de dibutyle ;
- les copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS;
- les homopolymères de styrène ;
- les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHODIA CHIMIE;
- les copolymères de styrène, de méthacrylate d'alkyle et d'acrylate d'alkyle ;
- les copolymères de styrène et de butadiène ;
- les copolymères de styrène, de butadiène et de vinylpyridine ;
- les copolymères d'acrylate d'alkyle et d'uréthanne ;
- les polyamides,
- les homopolymères et copolymères de vinyllactame.

Les groupes alkyle des polymères non ioniques mentionnés ci-dessus ont, de préférence, de 1 à 6 atomes de carbone.

Selon la présente invention, les polymères fixants non-ioniques à motifs vinyllactames peuvent être ceux décrits dans les brevets US 3 770 683, US 3 929 735, US 4 521 504, US 5 158 762, US 5 506 315 et dans les demandes de brevet WO 94/121148, WO 96/06592 et WO 96/10593. Ils peuvent se présenter sous forme pulvérulente ou sous forme de solution ou de suspension.

Les homopolymères ou copolymères à motifs vinyllactame comprennent des motifs de formule (IX): 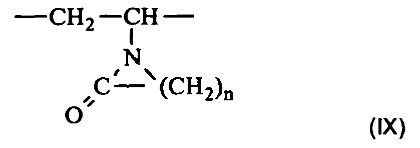 dans laquelle n est indépendamment 3, 4 ou 5.

La masse moléculaire en nombre des polymères à motifs vinyllactames est généralement supérieure à environ 5 000, de préférence comprise entre 10 000 et 1 000 000 environ, plus préférentiellement comprise entre 10 000 et 100 000 environ.

Parmi ces polymères fixants, on peut citer les polyvinylpyrrolidones telles que celles commercialisées sous la dénomination Luviskol^{®} K30 par la société BASF ; les polyvinylcaprolactames tels que ceux commercialisés sous la dénomination Luviskol^{®} PLUS par la société BASF ; les copolymères poly(vinylpyrrolidone/acétate de vinyle) tels que ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) tels que, par exemple, ceux commercialisés sous la dénomination Luviskol^{®} VAP 343 par la société BASF.

Les polymères fixants selon l'invention peuvent aussi être choisis parmi les polyuréthanes non ioniques ou anioniques éventuellement siliconés.

La teneur en polymère conditionneur ou en polymère fixant varie avantageusement de 0,01 à 20 % en poids, par rapport au poids de la composition tinctoriale, et de préférence de 0,1 à 5 % en poids, par rapport au poids de la composition tinctoriale.

La composition peut en outre comprendre au moins un additif choisi parmi les tensioactifs, choisis parmi les espèces non ioniques, anioniques, cationiques, amphotères ou zwittérioniques.

A titre d'exemple de tensioactifs anioniques utilisables, seuls ou mélanges, on peut citer notamment les sels des composés suivants, en particulier les sels de métaux alcalins ou alcalino-terreux, notamment le sodium, le magnésium ; les sels d'ammonium, les sels d'amines, les sels d'aminoalcools : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates.

On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates, les alkylsulfosuccinamates ; les acyliséthionates et les N-acyltaurates, le radical alkyle ou acyle des composés précités est de préférence en C₁₂-C₂₀, et le radical aryle désigne de préférence un groupement phényle ou benzyle.

Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle est en C₈-C₂₀.

On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

A titre d'exemple de tensioactifs non ioniques, on peut citer entre autres les alcools, les alpha-diols, les alkylphénols polyéthoxylés et/ou polypropoxylés, en C₈-C₁₈, le nombre de groupements oxyde d'éthylène et/ou oxyde de propylène étant compris entre 2 et 50.

On peut également citer les copolymères d'oxyde d'éthylène et d'oxyde de propylène, les condensats d'oxyde d'éthylène et d'oxyde de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

Pour ce qui a trait aux tensioactifs amphotères ou zwittérioniques, on peut mentionner sans avoir l'intention de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂-CONHCH₂CH₂-N(R₃)(R₄)(CH₂COO⁻)

dans laquelle :R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₂'-CONHCH₂CH₂-N(B)(C)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂-CHOH-SO₃H
R₂' désigne un radical alkyle d'un acide R₉-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid, disodium cocoamphocarboxy ethyl hydroxypropyl sulfonate.

A titre d'exemples on peut citer le cocoamphodiacetate commercialisé sous la dénomination commerciale MIRANOL^{®} C2M concentré par la société Rhodia Chimie.

Parmi les tensioactifs cationiques on peut citer en particulier les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique ; les mono- et di- esters quaternaires appelés esterquat.

La teneur en agents tensioactifs, lorsqu'ils sont présents, représente plus particulièrement de 0,001 à 30 % en poids et de préférence de 0,05 à 30 % en poids, par rapport au poids de la composition tinctoriale.

La composition tinctoriale peut comprendre en outre, au moins un colorant direct additionnel différent du composé de formule (I), choisi parmi les espèces non ioniques ou ioniques, et de préférence non ioniques ou cationiques.

De manière habituelle, le colorant direct additionnel peut être choisi parmi les colorants acridines, acridones, anthranthrones, anthrapyrimidines, anthraquinones, azines, azos, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines et notamment azacarbocyanine, diazacarbocyanine, diazahémicyanine, hémicyanine, tétraazacarbocyanine), diazines, dicétopyrrolopyrroles, dioxazines, diphénylamines, diphénylméthanes, dithiazines, flavanthrones, flavones, fluorindines, formazans, hydrazones, hydroxycétones, indamines, indanthrones, indigoides, indophénols, indoanilines, isoindolines, isoindolinones, isoviolanthrones, lactones, méthines, naphthalimides, naphthanilides, naphtholactames, naphthoquinones, nitro, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes, seuls ou en mélanges.

S'ils sont présents dans la composition tinctoriale, la teneur en colorant(s) direct(s) additionnel(s) différent(s) des composés de formule (I) représente de 0,0005 à 12 % en poids par rapport au poids de la composition tinctoriale, et de préférence de 0,005 à 6 % en poids par rapport au poids de la composition tinctoriale.

Comme variante de l'invention, la composition tinctoriale mise en oeuvre, peut comprendre au moins une base d'oxydation éventuellement associée à un ou plusieurs coupleurs.

La base d'oxydation peut être choisie parmi les composés classiques dans le domaine de la coloration.

A titre d'exemples, on peut citer entre autres les o-phénylènediamines, les p-phénylènediamines, les bases doubles, les o-aminophénols, les p-aminophénols, les bases hétérocycliques, leurs sels d'addition avec un acide, ainsi que leurs mélanges.

Si la composition tinctoriale comprend une ou plusieurs bases d'oxydation, la teneur en ce type de composé représente habituellement de 0,0005 à 12% en poids par rapport au poids de la composition, et de préférence de 0,005 à 8% en poids par rapport au poids de la composition.

Parmi les coupleurs utilisables en association avec une ou plusieurs bases d'oxydation, on peut mentionner par exemple les m-aminophénols, les m-phénylènediamines, les m-diphénols, les naphtols, les coupleurs hétérocycliques, leurs sels d'addition avec un acide, ainsi que leurs mélanges.

Au cas où ils sont présents dans la composition tinctoriale, leur teneur représente en général de 0,0001 à 10% en poids par rapport au poids de la composition, et de préférence de 0,005 à 5% en poids par rapport au poids de la composition.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut également comprendre divers adjuvants utilisés classiquement, tels que des agents épaississants minéraux, des agents antioxydants, des agents de pénétration, des parfums, des tampons, des agents dispersants, des agents de type ester, des huiles et des cires végétales, des huiles minérales, des agents filmogènes, des céramides, des vitamines ou provitamines, des agents conservateurs, des agents stabilisants, des agents opacifiants ou matifiants comme le dioxyde de titane, des charges minérales, comme les argiles, les silices notamment pyrogénées à caractère hydrophile ou hydrophobe, des polymères liants tels que la vinylpyrrolidone, des filtres solaires, etc.

Les adjuvants cités ci-dessus sont en général présents en quantité comprise pour chacun d'eux entre 0,01 et 20% en poids par rapport au poids de la composition.

Le milieu de la composition tinctoriale est un milieu cosmétiquement acceptable.

Il est constitué, de manière avantageuse, par de l'eau ou par un mélange d'eau et d'un ou plusieurs solvants organiques.

Parmi les solvants organiques usuels, on peut citer notamment les monoalcools ou les diols, linéaire ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants organiques décrits ci-dessus, s'ils sont présents, représentent habituellement de 1 à 40 % en poids, plus préférentiellement de 5 à 30 % en poids, par rapport au poids total de la composition.

Notons que la composition peut se trouver sous la forme d'une lotion épaissie ou non, d'une crème, d'un gel, ou de toute autre forme appropriée pour l'application ultérieure de cette composition.

La composition tinctoriale mise en oeuvre dans le cadre de l'invention peut éventuellement comprendre au moins un agent oxydant.

Habituellement, l'agent oxydant est choisi parmi le peroxyde d'hydrogène, les peroxydes de métaux alcalins ou alcalino-terreux, comme le sodium, le potassium, le magnésium ; le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges. L'agent oxydant peut aussi être choisi parmi les enzymes telles que les peroxydases et les oxydoréductases à deux ou à quatre électrons. De préférence, l'agent oxydant est le peroxyde d'hydrogène.

S'il est présent, la teneur en agent oxydant représente de 0,001 à 20% en poids par rapport au poids de la composition prête à l'emploi.

Si la composition mise en oeuvre dans le procédé selon l'invention comprend un agent oxydant, alors cette composition, appelée aussi composition prête à l'emploi, résulte plus particulièrement du mélange de la composition décrite auparavant dépourvue d'agent oxydant, avec une composition comprenant au moins un tel agent (cette composition est alors plus particulièrement appelée composition oxydante). Dans ce cas, le mélange est réalisé avant l'application de la composition prête à l'emploi sur les fibres à traiter.

Le milieu de la composition oxydante précitée est avantageusement de l'eau ou un mélange d'eau et de solvant organique.

Enfin, la composition oxydante peut comprendre les additifs usuels dans le domaine, comme des agents tensioactifs, des agents épaississants, des agents antioxydants, des parfums, des agents dispersants, des agents de conditionnement, des agents séquestrants, des agents conservateurs, etc.

Précisons que les listes de solvants et d'additifs, de même que leurs teneurs, indiquées dans le cadre de la description de la composition tinctoriale restent valables et l'on peut s'y reporter.

Le pH de la composition tinctoriale mise en oeuvre dans le procédé selon l'invention est généralement compris entre 5 et 11, et de préférence entre 6 et 8,5.

Il peut être ajusté à la valeur désirée au moyen d'agents acidifiants ou alcalinisants.

Parmi les agents acidifiants, on peut citer, à titre d'exemples, les acides minéraux ou organiques comme l'acide chlorhydrique, l'acide orthophosphorique, l'acide sulfurique, l'acide acétique.

Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 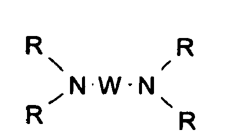 dans laquelle W est un reste propylène éventuellement substitué par un groupement hydroxyle ou un radical alkyle en C₁-C₆ ; les radicaux R, identiques ou différents, représentent un atome d'hydrogène, un radical alkyle en C₁-C₆ ou hydroxyalkyle en C₁-C₆.

Le procédé selon l'invention consiste plus particulièrement à mettre en oeuvre les étapes suivantes :
a) on met en contact les fibres, sèches ou humides, avec une composition comprenant au moins un composé de formule (I), pendant une durée suffisante pour le développement de la coloration,
b) on rince éventuellement les fibres,
c) éventuellement lave les fibres et on les rince,
d) on sèche les fibres ou on les laisse sécher.

Selon une première variante du procédé, la composition appliquée sur les fibres ne comprend pas d'agent oxydant.

Cette variante est particulièrement appropriée lorsque la composition ne comprend que le ou les composés de formule (I) et éventuellement un ou plusieurs colorants directs additionnels.

Dans le cadre de cette variante, il est envisageable de ne pas rincer ni laver au shampooing, les fibres une fois traitées.

De préférence, on procèdera toutefois à un tel rinçage, et éventuellement au lavage des fibres avec un shampooing.

Selon une deuxième variante du procédé, la composition appliquée sur les fibres est une composition comprenant au moins un agent oxydant.

Cette variante du procédé est appropriée pour toute composition tinctoriale mise en oeuvre, que celle-ci ne comprenne que des composés de formule (I) et éventuellement un ou plusieurs colorants directs additionnels, ou qu'elle comprenne, en plus du ou des composés de formule (I), une ou plusieurs bases d'oxydation et éventuellement un ou plusieurs coupleurs.

Conformément à cette deuxième variante, une première possibilité consiste à appliquer la composition obtenue par mélange extemporané avant l'application sur les fibres, de la composition tinctoriale dépourvue d'agent oxydant, avec une composition oxydante.

Selon cette possibilité, il peut être avantageux de stocker sous forme séparée, d'une part, la composition tinctoriale dépourvue d'agent oxydant, et d'autre part, une composition oxydante.

Une seconde possibilité consiste à remplacer l'étape a) par l'application successive, dans un ordre ou dans l'autre, et avec ou sans rinçage intermédiaire, de la composition tinctoriale dépourvue d'agent oxydant et de la composition oxydante, ou bien encore consiste à appliquer ces deux compositions simultanément.

Dans cette deuxième variante, une fois l'étape a) terminée, on peut éventuellement rincer les fibres (étape b), et éventuellement les laver avec un shampooing. De préférence, on rince et on lave les fibres.

Enfin, on sèche lesdites fibres ou bien encore on les laisse sécher, par exemple entre 20 et 220°C.

Il est à noter que la durée nécessaire au développement de la coloration de l'étape a) est en général comprise entre 1 à 60 minutes, et plus préférentiellement entre 5 et 45 minutes.

Par ailleurs, de manière classique l'étape a) du procédé est effectuée à une température comprise entre 15 et 220°C, de préférence à une température comprise entre 15 et 40°C.

### EXEMPLE 1:

On a préparé la composition 1 de teinture suivante :

| | |
|---|---|
| Chlorure de 7,14-Bis-(2-hydroxy-ethyl)-5-methyl-7,14-dihydro- | 0,39 g |
| quinoxalino[2,3-b]phenazin-5-ium (10⁻³ mole) (*) | |
| Alcool benzylique | 4,0 g |
| Polyéthylèneglycol 60E | 6,0 g |
| Hydroxyéthylcellulose | 0,7 g |
| Alkylpolyglucoside en solution aqueuse à 60% M.A** | 4,5 g M.A |
| Tampon phosphate q.s. pH | 7 |
| Eau déminéralisée q.s.p. | 100 g |

| | |
|---|---|
| *composé de formule suivante : 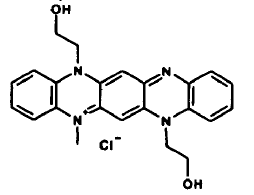 ** Matière Active | |

On a appliqué la composition ci-dessus sur des mèches de cheveux gris naturels ou permanentés à 90% de blancs et on a laissé poser pendant 20 minutes.

Après rinçage à l'eau courante et séchage, les cheveux ont été teints dans une nuance bleue.

### EXEMPLE 2:

On a préparé la composition 2 de teinture suivante :

| | |
|---|---|
| Chlorure de 1H-Benzo[ij]quinolizinium, 9-[bis(2,3,6,7-tetrahydro-1H,5H-benzo[ij]quinolizin-9-yl)methylene]-2,3,5,6,7,9-hexahydro (10⁻³ mole) | 0,39 g |
| Diéthanolamide olëique | 3 g |
| Acide laurique | 1 g |
| Monoéthyléther de l'éthylèneglycol | 5 g |
| Hydroxyéthylcellulose | 2 g |
| 2-amino-2-méthyl-1-propanol q.s. pH | 9,5 |
| Eau déminéralisée q.s.p.. | 100 g |

On a appliqué la composition ci-dessus sur des mèches de cheveux gris naturels ou permanentés à 90% de blancs et on a laissé poser pendant 30 minutes.

Après rinçage à l'eau courante et séchage, les cheveux ont été teints dans une nuance bleu.

### EXEMPLES 3

On a préparé les compositions 3(A) à 3(D), conformes à l'invention, suivantes (teneurs en grammes) :

| COMPOSITION | A | B | C | D |
|---|---|---|---|---|
| | | | | |
| Paratoluylènediamine | 0,25 | - | - | - |
| Para-aminophénol | 0,30 | 0,50 | 0,15 | - |
| Paraphénylènediamine | - | 0,20 | | 0,30 |
| | | | | |
| 5-N-(β-hydroxyéthyl)amino 2-méthyl phénol | 0,5 | 0,8 | 0,17 | - |
| 5-amino 2-méthyl phénol | - | - | - | 0,30 |
| | | | | |
| Colorant cationique de structure (1) | 0,39 | 0,39 | 0,39 | 0,39 |
| | | | | |
| Support de teinture commun (*) | (*) | (*) | (*) | (*) |
| Eau q.s.p. | 100 g | 100 g | 100 g | 100 g |

| | | | | |
|---|---|---|---|---|
| (*) support de teinture commun : - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3,0 g - Amine oléique à 2 moles d'oxyde d'éthylène vendue sous la dénomination commerciale ETHOMEEN 012 par la société AKZO 7,0 g - Laurylamino succinamate de diéthylaminopropyle, sel de sodium, à 55 % de M.A. 3,0 g M.A. - Alcool oléique 5,0 g - Diéthanolamide d'acide oléique 12,0 g - Propylèneglycol 3,5 g - Alcool éthyliques 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - Antioxydant, séquestrant q.s. - Parfum, conservateur q.s. - Ammoniaque à 20 % de NH₃ 10,0 g | | | | |

Au moment de l'emploi, on a mélangé chacune de ces compositions 3(A) à 3(D) avec une quantité égale d'une composition (B) constituée par une solution de peroxyde d'hydrogène à 20 volumes (6 % en poids).

Chaque composition résultante (composition prête à l'emploi conforme à l'invention) a été appliquée pendant 30 minutes sur des mèches de cheveux gris naturels à 90 % de blancs.

Les mèches de cheveux ont ensuite été rincées, lavées avec un shampooing standard puis séchées.

Les mèches de cheveux ont été teintes dans les nuances figurant dans le tableau ci-dessous :

| **EXEMPLE** | **NUANCE OBTENUE** |
|---|---|
| COMPOSITION A | Blond foncé à reflet bleu |
| COMPOSITION B | Blond à reflet bleu |
| COMPOSITION C | Blond clair à reflet bleu |
| COMPOSITION D | Blond à reflet bleu |

Les nuances obtenues ont présenté une très bonne ténacité aux shampooings ultérieurs.

### EXEMPLE 4

On a préparé la composition 4 suivante :
- 1,4-diamino benzène 0,40 g
- 5-amino 2-méthyl phénol 0,45 g
- Support de teinture commun tel que décrit précédemment pour l'exemple 3 (*)
- Eau déminéralisée q.s.p. 100 g

On a préparé la composition 4' suivante :
- Colorant cationique Chlorure de 7,14-Bis-(2-hydroxy-ethyl)-5-méthyl-7,14-dihydro-quinoxalino[2,3-b]phenazin-5-ium 4 g
- Polyammonium quaternaire vendu sous la dénomination commerciale CELQUAT SC-240 par la société National Starch 10 g
- Sciure de bois q.s.p. 100 g

Au moment de l'emploi, on a mélangé une partie en poids de la composition 4 ci-dessus avec 0,1 partie en poids de la composition 4' et avec une partie en poids d'une composition (B) constituée par une solution de peroxyde d'hydrogène à 20 volumes (6 % en poids).

La composition résultante a été appliquée pendant 30 minutes sur des mèches de cheveux gris naturels à 90 % de blancs. Les cheveux ont ensuite été rincées, lavées avec un shampooing standard puis séchées.

Les cheveux ont été teints dans une nuance châtain clair à reflet bleu résistant très bien aux shampooings ultérieurs.

## Revendications

1. Utilisation pour la teinture de fibres kératiniques, notamment humaines, d'une composition tinctoriale comprenant dans un milieu approprié pour la teinture, à titre de colorant direct, au moins un composé de formule (I) suivante : 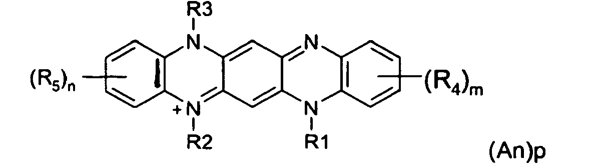 Formule dans laquelle :
R₁, R₂, R₃, identiques ou différents, représentent un radical alkyle en C₁-C₂₄, linéaire ou ramifié ; un radical aryle en C₆-C₃₀ ; un radical arylalkyle dans lequel la partie aryle est en C₆-C₃₀ et la partie alkyle, linéaire ou ramifiée est en C₁-C₂₄ ;
R₄, R₅, identiques ou non, représentent un atome d'hydrogène ; un atome d'halogène ; un radical hydroxy ; un radical alkyle en C₁-C₂₄, linéaire ou ramifié ;
un radical alcoxy en C₁-C₂₄, linéaire ou ramifié ; un groupement monohydroxy alcoxy dont la partie alkyle est en C₁-C₆, linéaire ou ramifiée ; un groupement polyhydroxy alcoxy, dans lequel la partie alkyle est en C₁-C₆, linéaire ou ramifiée ;
un groupement amino substitué ou non par un ou plusieurs radicaux alkyle en C₁-C₆, identiques ou non, linéaires ou ramifiés, substitués ou non par un ou plusieurs groupement hydroxyle ; un groupement thiol ; un groupement alkylthio dans lequel la partie alkyle est en C₁-C₆, linéaire ou ramifiée ; un groupement carboxylique sous forme acide ou salifiée (avec un métal alcalin ou un ammonium substitué ou non) ; un groupement alcoxycarbonyle dont la partie alkyle est en C₁-C₆, linéaire ou ramifiée ; un groupement alkylamide dont la partie alkyle est en C₁-C₆, linéaire ou ramifiée ; un groupement alkylcarbamyle dont la partie alkyle est en C₁-C₆, linéaire ou ramifiée ; un groupement cyano ; un groupement nitro ; un groupement sulfonyle ; un groupement alkylsulfonyle dont la partie alkyle est en C₁-C₆, linéaire ou ramifiée ; un groupement alkylsulfonylamido dont la partie alkyle est en C₁-C₆, linéaire ou ramifiée ; un radical aryle en C₆-C₃₀ éventuellement substitué par un ou plusieurs radicaux alkyle en C₁-C₆ ; un radical arylalkyle dont la partie aryle est en C₆-C₃₀, éventuellement substituée par un ou plusieurs radicaux alkyle en C₁-C₆ et la partie alkyle, linéaire ou ramifiée, est en C₁-C₂₄,
les radicaux alkyle ou aryle tels quels ou les parties alkyle des radicaux composés étant éventuellement substitués par un ou plusieurs groupements choisis parmi un groupement hydroxyle, un groupement -SO₃⁻ , un groupement -COO⁻, un atome d'halogène, un groupement alcoxy en C₁-C₆ ; un groupement monohydroxy alcoxy dont la partie alkyle en C₁-C₆, linéaire ou ramifiée ; un groupement polyhydroxy alcoxy, dans lequel la partie alkyle est en C₁-C₆, linéaire ou ramifiée ; un groupement amino substitué ou non par un ou plusieurs radicaux alkyles en C₁-C₆, identiques ou non, linéaires ou ramifiés, substitué ou non par un ou plusieurs groupement hydroxyle
n, m, indépendamment l'un de l'autre, représentent un nombre entier compris entre 0 et 4 ;
An représente un anion ou un mélange d'anions cosmétiquement acceptables ; p valant 0 ou 1, afin de respecter l'électroneutralité du composé.

2. Utilisation selon la revendication précédente, **caractérisée en ce que**, dans la formule (I) les radicaux R₁, R₂, R₃, identiques ou différents, représentent un radical alkyle en C₁-C₆ ; un radical phényle ; un radical arylalkyle dans lequel la partie aryle est en C₆ et la partie alkyle est en C₁-C₆ ; les radicaux alkyle, phényle, arylalkyle étant éventuellement substitués par un ou plusieurs groupements choisis parmi les radicaux hydroxyle, -SO₃⁻,-COO⁻, halogène, alcoxy en C₁-C₆, amino, (di)alkylamino dont la partie alkyle est en C₁-C₆, (di)hydroxyalkylamino dont la partie alkyle est en C₁-C₆.

3. Utilisation selon la revendication précédente, **caractérisée en ce que**, dans la formule (I), les radicaux R₁, R₂, R₃, identiques ou différents, représentent les radicaux méthyle, éthyle, méthoxy, hydroxyéthyle, 3-sulfopropyle, phényle.

4. Utilisation selon l'une quelconque des revendications précédentes, **caractérisée en ce que**, dans la formule (1), R₄, R₅, identiques ou non, représentent un atome d'hydrogène ; un atome de chlore ; un radical amino ; un radical (di)alkylamino dont la partie alkyle est en C₁-C₆ ; un radical (di)hydroxyalkylamino dont la partie alkyle est en C₁-C₆ ; un radical hydroxy ; un radical alkyle en C₁-C₆, linéaire ou ramifié, un radical alcoxy en C₁-C₆, linéaire ou ramifié, lesdits radicaux alkyle ou alcoxy étant éventuellement substitué par un ou plusieurs groupements choisis parmi les radicaux hydroxy, halogène, alcoxy en C₁-C₆, amino, (di)alkylamino dont la partie alkyle est en C₁-C₆, (di)hydroxyalkylamino dont la partie alkyle est en C₁-C₆.

5. Utilisation selon la revendication précédente, **caractérisée en ce que**, dans la formule (I), R₄, R₅, identiques ou non, représentent un atome d'hydrogène, un radical choisi parmi les groupements méthyle, éthyle, méthoxy, éthoxy, hydroxy, amino, diméthylamino, dihydroxyéthylamino, un atome de chlore.

6. Utilisation selon l'une quelconque des revendications précédentes, **caractérisée en ce que** la teneur en composé de formule (I) représente 0,001 à 20 % en poids de la composition tinctoriale, de préférence de 0,01 à 10 % en poids par rapport au poids de la composition tinctoriale.

7. Utilisation selon l'une quelconque des revendications précédentes, **caractérisée en ce que** la composition comprend au moins un colorant direct additionnel différent du composé de formule (I), choisis parmi les espèces non ioniques ou ioniques, et de préférence non ioniques ou cationiques.

8. Utilisation selon la revendication précédente, **caractérisée en ce que** le colorant direct additionnel est choisi parmi les colorants acridines, acridones, anthranthrones, anthrapyrimidines, anthraquinones, azines, azos, azométhines, benzanthrones, benzimidazoles, benzimidazolones, benzindoles, benzoxazoles, benzopyranes, benzothiazoles, benzoquinones, bisazines, bis isoindolines, carboxanilides, coumarines, cyanines, diazines, dicétopyrrolopyrroles, dioxazines, diphénylamines, diphénylméthanes, dithiazines, flavanthrones, flavones, fluorindines, formazans, hydrazones, hydroxycétones, indamines, indanthrones, indigoides, indophénols, indoanilines, isoindolines, isoindolinones, isoviolanthrones, lactones, méthines, naphthalimides, naphthanilides, naphtholactames, naphthoquinones, nitro, oxadiazoles, oxazines, périlones, périnones, pérylènes, phénazines, phénothiazines, phthalocyanine, polyènes/caroténoides, porphyrines, pyranthrones, pyrazolanthrones, pyrazolones, pyrimidinoanthrones, pyronines, quinacridones, quinolines, quinophthalones, squaranes, stilbènes, tétrazoliums, thiazines, thioindigo, thiopyronines, triarylméthanes, xanthènes, seuls ou en mélanges.

9. Utilisation selon la revendication précédente, **caractérisée en ce que** la teneur en colorant(s) direct(s) additionnel(s) différent(s) des composés de formule (I), représente de 0,0005 à 12 % en poids par rapport au poids de la composition tinctoriale, et de préférence de 0,005 à 6 % en poids par rapport au poids de la composition tinctoriale.

10. Utilisation selon l'une quelconque des revendications précédentes, **caractérisée en ce que** la composition tinctoriale comprend au moins un agent oxydant.

11. Utilisation selon la revendication précédente, **caractérisée en ce que** l'agent oxydant est choisi parmi le peroxyde d'hydrogène, le peroxyde d'urée, les bromates ou ferricyanures de métaux alcalins, les persels tels que les perborates et les persulfates, les enzymes, seuls ou en mélanges.

12. Procédé de teinture de fibres kératiniques, notamment humaines, en particulier les cheveux, dans lequel on met en oeuvre les étapes suivantes :
a) on met en contact lesdites fibres, sèches ou humides, avec une composition comprenant au moins un composé de formule (I) telle que décrite dans l'une quelconque des revendications précédentes, pendant une durée suffisante pour le développement de la coloration,
b) on rince éventuellement les fibres,
c) éventuellement lave les fibres et on les rince,
d) on sèche les fibres ou on les laisse sécher.

13. Composition tinctoriale comprenant dans un milieu approprié pour la teinture des fibres kératiniques, en particulier humaines, à titre de colorant direct, au moins un composé de formule (I) suivante : 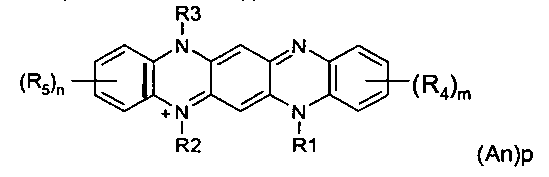 Formule dans laquelle :
R₁, R₂, R₃, identiques ou différents, représentent un radical alkyle en C₁-C₂₄, linéaire ou ramifié ; un radical aryle en C₆-C₃₀ ; un radical arylalkyle dans lequel la partie aryle est en C₆-C₃₀ et la partie alkyle, linéaire ou ramifiée est en C₁-C₂₄ ;
R₄, R₅, identiques ou non, représentent un atome d'hydrogène ; un atome d'halogène ; un radical hydroxy ; un radical alkyle en C₁-C₂₄, linéaire ou ramifié ;
un radical alcoxy en C₁-C₂₄, linéaire ou ramifié ; un groupement monohydroxy alcoxy dont la partie alkyle est en C₁-C₆, linéaire ou ramifiée ; un groupement polyhydroxy alcoxy, dans lequel la partie alkyle est en C₁-C₆, linéaire ou ramifiée ;
un groupement amino substitué ou non par un ou plusieurs radicaux alkyle en C₁-C₆, identiques ou non, linéaires ou ramifiés, substitués ou non par un ou plusieurs groupement hydroxyle ; un groupement thiol ; un groupement alkylthio dans lequel la partie alkyle est en C₁-C₆, linéaire ou ramifiée ; un groupement carboxylique sous forme acide ou salifiée (avec un métal alcalin ou un ammonium substitué ou non) ; un groupement alcoxycarbonyle dont la partie alkyle est en C₁-C₆, linéaire ou ramifiée ; un groupement alkylamide dont la partie alkyle est en C₁-C₆, linéaire ou ramifiée ; un groupement alkylcarbamyle dont la partie alkyle est en C₁-C₆, linéaire ou ramifiée ; un groupement cyano ; un groupement nitro ; un groupement sulfonyle ; un groupement alkylsulfonyle dont la partie alkyle est en C₁-C₆, linéaire ou ramifiée ; un groupement alkylsulfonylamido dont la partie alkyle est en C₁-C₆, linéaire ou ramifiée ; un radical aryle en C₆-C₃₀ éventuellement substitué par un ou plusieurs radicaux alkyle en C₁-C₆ ; un radical arylalkyle dont la partie aryle est en C₆-C₃₀, éventuellement substituée par un ou plusieurs radicaux alkyle en C₁-C₆ et la partie alkyle, linéaire ou ramifiée, est en C₁-C₂₄,
les radicaux alkyle ou aryle tels quels ou les parties alkyle des radicaux composés étant éventuellement substitués par un ou plusieurs groupements choisis parmi un groupement hydroxyle, un groupement -SO₃⁻ , un groupement -COO⁻, un atome d'halogène, un groupement alcoxy en C₁-C₆; un groupement monohydroxy alcoxy dont la partie alkyle en C₁-C₆, linéaire ou ramifiée ; un groupement polyhydroxy alcoxy, dans lequel la partie alkyle est en C₁-C₆, linéaire ou ramifiée ; un groupement amino substitué ou non par un ou plusieurs radicaux alkyles en C₁-C₆, identiques ou non, linéaires ou ramifiés, substitué ou non par un ou plusieurs groupement hydroxyle,
n, m, indépendamment l'un de l'autre, représentent un nombre entier compris entre 0 et 4,
An représente un anion ou un mélange d'anions cosmétiquement acceptables ; p valant 0 ou 1, afin de respecter l'électroneutralité du composé ;
et au moins un additif choisi parmi les polymères à l'exception de polymères tensioactifs dérivés d'éthylène oxy-alkylphénol.

14. Composition selon la revendication précédente, **caractérisée en ce que**, dans la formule (I) les radicaux R₁, R₂, R₃, identiques ou différents, représentent un radical alkyle en C₁-C₆ ; un radical phényle ; un radical arylalkyle dans lequel la partie aryle est en C₆ et la partie alkyle est en C₁-C₆; les radicaux alkyle, phényle, arylalkyle étant éventuellement substitués par un ou plusieurs groupements choisis parmi les radicaux hydroxy, halogène, alcoxy en C₁-C₆, amino, (di)alkylamino dont la partie alkyle est en C₁-C₆, (di)hydroxyalkylamino dont la partie alkyle est en C₁-C₆.

15. Composition selon la revendication précédente, **caractérisée en ce que**, dans la formule (I), les radicaux R₁, R₂, R₃, identiques ou différents, représentent De préférence, les radicaux R₁, R₂, R₃, identiques ou différents, représentent les radicaux méthyle, éthyle, méthoxy, hydroxyéthyle, 3-sulfopropyle, phényle.

16. Composition selon l'une quelconque des revendications 14 ou 15, **caractérisée en ce que**, dans la formule (I), R₄, R₅, identiques ou non, représentent un atome d'hydrogène ; un atome de chlore ; un radical amino ; un radical (di)alkylamino dont la partie alkyle est en C₁-C₆ ; un radical (di)hydroxyalkylamino dont la partie alkyle est en C₁-C₆ ; un radical hydroxy ; un radical alkyle en C₁-C₆, linéaire ou ramifié, un radical alcoxy en C₁-C₆, linéaire ou ramifié, lesdits radicaux alkyle ou alcoxy étant éventuellement substitué par un ou plusieurs groupements choisis parmi les radicaux hydroxy, halogène, alcoxy en C₁-C₆, amino, (di)alkylamino dont la partie alkyle est en C₁-C₆, (di)hydroxyalkylamino dont la partie alkyle est en C₁-C₆.

17. Composition selon la revendication précédente, **caractérisée en ce que**, dans la formule (I), R₄, R₅, identiques ou non, représentent un atome d'hydrogène, un radical choisi parmi les groupements méthyle, éthyle, méthoxy, éthoxy, hydroxy, amino, diméthylamino, dihydroxyéthylamino, un atome de chlore.

18. Composition selon l'une quelconque des revendications 13 à 17, **caractérisée en ce que** la teneur en composé de formule (I) représente 0,001 à 20 % en poids de la composition tinctoriale, de préférence de 0,01 à 10 % en poids par rapport au poids de la composition tinctoriale.

19. Composition selon l'une quelconque des revendications 13 à 18, **caractérisée en ce que** la composition comprend un additif choisi parmi les agents tensioactifs de nature non ionique, anionique, cationique ou amphotère.

20. Composition selon l'une quelconque des revendications 13 à 19, **caractérisée en ce que** le polymère est de nature non ionique, cationique, anionique ou amphotère.

21. Composition selon la revendication précédente, **caractérisée en ce que** la teneur en agents tensioactifs représente plus particulièrement de 0,001 à 30 % en poids et de préférence de 0,05 à 30 % en poids, par rapport au poids de la composition tinctoriale.

22. Composition selon l'une quelconque des revendications 13 à 21, **caractérisée en ce que** le polymère est un polymère épaississant, associatif ou non.

23. Composition selon la revendication précédente, **caractérisée en ce que** la teneur en en polymère(s) épaississant(s) associatif(s) ou non, varie de 0,01 à 10% en poids, plus particulièrement de 0,1 à 5% en poids, par rapport au poids de la composition tinctoriale.

24. Composition selon l'une quelconque des revendications 13 à 23, **caractérisée en ce que** le polymère est un polymère conditionneur ou un polymère fixant.

25. Composition selon la revendication précédente, **caractérisée en ce que** la teneur en polymère conditionneur ou en polymère fixant varie de 0,01 à 20 % en poids, par rapport au poids de la composition tinctoriale, et de préférence de 0,1 à 5 % en poids, par rapport au poids de la composition tinctoriale.

26. Composé de formule (I') suivante : 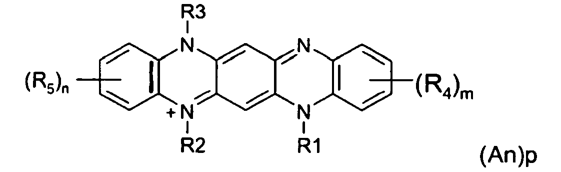 Formule dans laquelle :
R₁, R₂, R₃, identiques ou différents, représentent un radical alkyle en C₁-C₂₄, linéaire ou ramifié ; un radical aryle en C₆-C₃₀ ; un radical arylalkyle dans lequel la partie aryle est en C₆-C₃₀ et la partie alkyle, linéaire ou ramifiée est en C₁-C₂₄ ; à la condition que les radicaux R₁, R₃, identiques ou différents, représentent des radicaux alkyle porteurs d'au moins un groupement hydroxyle ;
R₄, R₅, identiques ou non, représentent un atome d'hydrogène ; un atome d'halogène ; un radical hydroxyle ; un radical alkyle en C₁-C₂₄, linéaire ou ramifié ; un radical alcoxy en C₁-C₂₄, linéaire ou ramifié ; un groupement monohydroxy alcoxy dont la partie alkyle est en C₁-C₆, linéaire ou ramifiée ; un groupement polyhydroxy alcoxy, dans lequel la partie alkyle est en C₁-C₆, linéaire ou ramifiée ; un groupement amino substitué ou non par un ou plusieurs radicaux alkyle en C₁-C₆, identiques ou non, linéaires ou ramifiés, substitués ou non par un ou plusieurs groupement hydroxyle ; un groupement thiol ; un groupement alkylthio dans lequel la partie alkyle est en C₁-C₆. linéaire ou ramifiée ; un groupement carboxylique sous forme acide ou salifiée (avec un métal alcalin ou un ammonium substitué ou non) ; un groupement alcoxycarbonyle dont la partie alkyle est en C₁-C₆, linéaire ou ramifiée ; un groupement alkylamide dont la partie alkyle est en C₁-C₆, linéaire ou ramifiée ; un groupement alkylcarbamyle dont la partie alkyle est en C₁-C₆, linéaire ou ramifiée ; un groupement cyano ; un groupement nitro ; un groupement sulfonyle ; un groupement alkylsulfonyle dont la partie alkyle est en C₁-C₆, linéaire ou ramifiée ; un groupement alkylsulfonylamido dont la partie alkyle est en C₁-C₆, linéaire ou ramifiée ; un radical aryle en C₆-C₃₀ éventuellement substitué par un ou plusieurs radicaux alkyle en C₁-C₆ ; un radical arylalkyle dont la partie aryle est en C₆-C₃₀, éventuellement substituée par un ou plusieurs radicaux alkyle en C₁-C₆ et la partie alkyle, linéaire ou ramifiée, est en C₁-C₂₄,
les radicaux alkyle ou aryle tels quels ou les parties alkyle des radicaux composés étant éventuellement substitués par un ou plusieurs groupements choisis parmi un groupement hydroxyle, un groupement -SO₃⁻, un groupement -COO⁻, un atome d'halogène, un groupement alcoxy en C₁-C₆; un groupement monohydroxy alcoxy dont la partie alkyle en C₁-C₆, linéaire ou ramifiée ; un groupement polyhydroxy alcoxy, dans lequel la partie alkyle est en C₁-C₆, linéaire ou ramifiée ; un groupement amino substitué ou non par un ou plusieurs radicaux alkyles en C₁-C₆, identiques ou non, linéaires ou ramifiés, substitué ou non par un ou plusieurs groupement hydroxyle,
n, m, indépendamment l'un de l'autre, représentent un nombre entier compris entre 0 et 4;
An représente un anion ou un mélange d'anions cosmétiquement acceptables ; p valant 0 ou 1, afin de respecter l'électroneutralité du composé à l'exception du composé suivant: 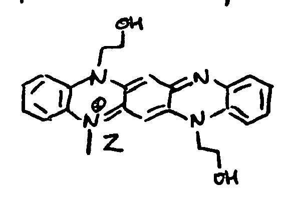 avec Z représentant un anion.

27. Composé selon la revendication 26, correspondant à l'une des formules (i) (ii) (iii) suivantes :

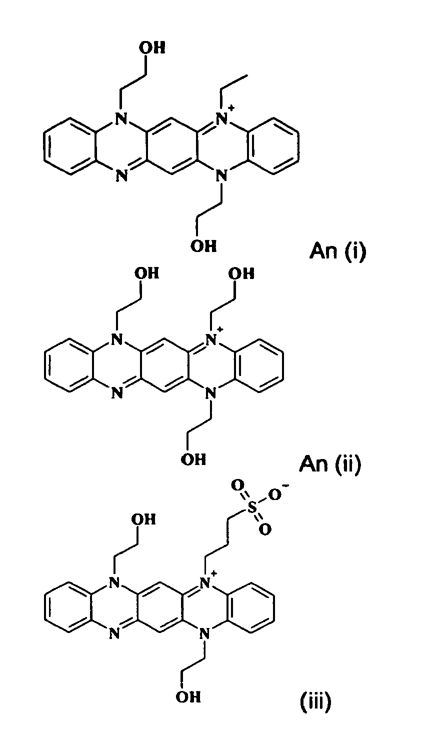

## Claims

1. Use for dyeing keratin fibres, especially human keratin fibres, of a dye composition comprising in a suitable dyeing medium, as direct dye, at least one compound of formula (I) below: 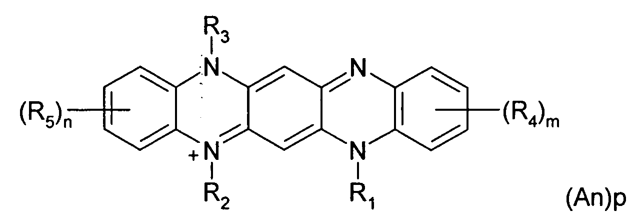 in which formula:
R₁, R₂ and R₃, which may be identical or different, represent a linear or branched C₁-C₂₄ alkyl radical; a C₆-C₃₀ aryl radical; an arylalkyl radical in which the aryl part is C₆-C₃₀ and the linear or branched alkyl part is C₁-C₂₄;
R₄ and R₅, which may be identical or different, represent a hydrogen atom; a halogen atom; a hydroxyl radical; a linear or branched C₁-C₂₄ alkyl radical; a linear or branched C₁-C₂₄ alkoxy radical; a monohydroxyalkoxy group in which the linear or branched alkyl part is C₁-C₆; a polyhydroxyalkoxy group in which the linear or branched alkyl part is C₁-C₆; an amino group optionally substituted with one or more identical or different, linear or branched C₁-C₆ alkyl radicals, optionally substituted with one or more hydroxyl groups; a thiol group; an alkylthio group in which the linear or branched alkyl part is C₁-C₆; a carboxylic group in acid or salified form (with an alkali metal or a substituted or unsubstituted ammonium); an alkoxycarbonyl group in which the linear or branched alkyl part is C₁-C₆; an alkylamide group in which the linear or branched alkyl part is C₁-C₆; or an alkylcarbamyl group in which the linear or branched alkyl part is C₁-C₆; a cyano group; a nitro group; a sulfonyl group; an alkylsulfonyl group in which the linear or branched alkyl part is C₁-C₆; an alkylsulfonylamido group in which the linear or branched alkyl part is C₁-C₆; a C₆-C₃₀ aryl radical optionally substituted with one or more C₁-C₆ alkyl radicals; an arylalkyl radical in which the aryl part is C₆-C₃₀, optionally substituted with one or more C₁-C₆ alkyl radicals and the linear or branched alkyl part is C₁-C₂₄,
the alkyl or aryl radicals per se or the alkyl parts of the compound radicals being optionally substituted with one or more groups chosen from a hydroxyl group, a -SO₃⁻ group, a -COO⁻ group, a halogen atom, a C₁-C₆ alkoxy group; a monohydroxyalkoxy group in which the linear or branched alkyl part is C₁-C₆; a polyhydroxyalkoxy group in which the linear or branched alkyl part is C₁-C₆; an amino group optionally substituted with one or more identical or different, linear or branched C₁-C₆ alkyl radicals, optionally substituted with one or more hydroxyl groups,
n and m, independently of each other, represent an integer between 0 and 4;
An represents a cosmetically acceptable anion or mixture of anions; p being 0 or 1, in order to respect the electrical neutrality of the compound.

2. Use according to the preceding claim, **characterized in that**, in formula (I), the radicals R₁, R₂ and R₃, which may be identical or different, represent a C₁-C₆ alkyl radical; a phenyl radical; an arylalkyl radical in which the aryl part is C₆ and the alkyl part is C₁-C₆; the alkyl, phenyl and arylalkyl radicals being optionally substituted with one or more groups chosen from hydroxyl, -SO₃⁻, -COO⁻, halogen, C₁-C₆ alkoxy, amino, (di)alkylamino in which the alkyl part is C₁-C₆, and (di)hydroxyalkylamino in which the alkyl part is C₁-C₆.

3. Use according to the preceding claim, **characterized in that**, in formula (I), the radicals R₁, R₂ and R₃, which may be identical or different, represent methyl, ethyl, methoxy, hydroxyethyl, 3-sulfopropyl or phenyl radicals.

4. Use according to any one of the preceding claims, **characterized in that**, in formula (I), R₄ and R₅, which may be identical or different, represent a hydrogen atom; a chlorine atom; an amino radical; a (di)alkylamino radical in which the alkyl part is C₁-C₆; a (di)hydroxyalkylamino radical in which the alkyl part is C₁-C₆; a hydroxyl radical; a linear or branched C₁-C₆ alkyl radical or a linear or branched C₁-C₆ alkoxy radical, the said alkyl or alkoxy radicals being optionally substituted with one or more groups chosen from hydroxyl, halogen, C₁-C₆ alkoxy, amino, (di)alkylamino in which the alkyl part is C₁-C₆, and (di)hydroxyalkylamino in which the alkyl part is C₁-C₆.

5. Use according to the preceding claim, **characterized in that**, in formula (I), R₄ and R₅, which may be identical or different, represent a hydrogen atom or a radical chosen from methyl, ethyl, methoxy, ethoxy, hydroxyl, amino, dimethylamino and dihydroxyethylamino radicals and a chlorine atom.

6. Use according to any one of the preceding claims, **characterized in that** the content of compound of formula (I) represents 0.001% to 20% by weight of the dye composition and preferably from 0.01% to 10% by weight relative to the weight of the dye composition.

7. Use according to any one of the preceding claims, **characterized in that** the composition comprises at least one additional direct dye other than the compound of formula (I), chosen from nonionic and ionic species, and preferably nonionic or cationic species.

8. Use according to the preceding claim, **characterized in that** the additional direct dye is chosen from acridine, acridone, anthranthrone, anthrapyrimidine, anthraquinone, azine, azo, azomethine, benzanthrone, benzimidazole, benzimidazolone, benzindole, benzoxazole, benzopyran, benzothiazole, benzoquinone, bisazine, bis-isoindoline, carboxanilide, coumarin, cyanin, diazin, diketopyrrolopyrrole, dioxazine, diphenylamine, diphenylmethane, dithiazine, flavanthrone, flavone, fluorindine, formazan, hydrazone, hydroxy ketone, indamine, indanthrone, indigoid, indophenol, indoaniline, isoindoline, isoindolinone, isoviolanthrone, lactone, methine, naphthalimide, naphthanilide, naphtholactam, naphthoquinone, nitro, oxadiazole, oxazine, perilone, perinone, perylene, phenazine, phenothiazine, phthalocyanin, polyene/carotenoid, porphyrin, pyranthrone, pyrazolanthrone, pyrazolone, pyrimidinoanthrone, pyronine, quinacridone, quinoline, quinophthalone, squarane, stilbene, tetrazolium, thiazine, thioindigo, thiopyronine, triarylmethane and xanthene dyes, alone or as mixtures.

9. Use according to the preceding claim, **characterized in that** the content of additional direct dye(s) other than the compounds of formula (I) represents from 0.0005% to 12% by weight relative to the weight of the dye composition, and preferably from 0.005% to 6% by weight relative to the weight of the dye composition.

10. Use according to any one of the preceding claims, **characterized in that** the dye composition comprises at least one oxidizing agent.

11. Use according to the preceding claim, **characterized in that** the oxidizing agent is chosen from hydrogen peroxide, urea peroxide, alkali metal bromates or ferricyanides, persalts such as perborates and persulfates, and enzymes, alone or as mixtures.

12. Process for dyeing keratin fibres, especially human keratin fibres, in particular the hair, in which the following steps are performed:
a) the said wet or dry fibres are placed in contact with a composition comprising at least one compound of formula (I) as described in any one of the preceding claims, for a time that is sufficient to develop the coloration,
b) the fibres are optionally rinsed,
c) the fibres are optionally washed and rinsed,
d) the fibres are dried or are left to dry.

13. Dye composition comprising, in a medium that is suitable for dyeing keratin fibres, in particular human keratin fibres, as direct dye, at least one compound of formula (I) below: 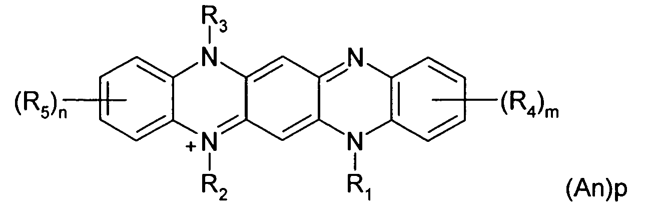 in which formula:
R₁, R₂ and R₃, which may be identical or different, represent a linear or branched C₁-C₂₄ alkyl radical; a C₆-C₃₀ aryl radical; an arylalkyl radical in which the aryl part is C₆-C₃₀ and the linear or branched alkyl part is C₁-C₂₄;
R₄ and R₅, which may be identical or different, represent a hydrogen atom; a halogen atom; a hydroxyl radical; a linear or branched C₁-C₂₄ alkyl radical; a linear or branched C₁-C₂₄ alkoxy radical; a monohydroxyalkoxy group in which the linear or branched alkyl part is C₁-C₆; a polyhydroxyalkoxy group in which the linear or branched alkyl part is C₁-C₆; an amino group optionally substituted with one or more identical or different, linear or branched C₁-C₆ alkyl radicals, optionally substituted with one or more hydroxyl groups; a thiol group; an alkylthio group in which the linear or branched alkyl part is C₁-C₆; a carboxylic group in acid or salified form (with an alkali metal or a substituted or unsubstituted ammonium); an alkoxycarbonyl group in which the linear or branched alkyl part is C₁-C₆; an alkylamide group in which the linear or branched alkyl part is C₁-C₆; or an alkylcarbamyl group in which the linear or branched alkyl part is C₁-C₆; a cyano group; a nitro group; a sulfonyl group; an alkylsulfonyl group in which the linear or branched alkyl part is C₁-C₆; an alkylsulfonylamido group in which the linear or branched alkyl part is C₁-C₆; a C₆-C₃₀ aryl radical optionally substituted with one or more C₁-C₆ alkyl radicals; an arylalkyl radical in which the aryl part is C₆-C₃₀, optionally substituted with one or more C₁-C₆ alkyl radicals and the linear or branched alkyl part is C₁-C₂₄,
the alkyl or aryl radicals per se or the alkyl parts of the compound radicals being optionally substituted with one or more groups chosen from a hydroxyl group, a -SO₃⁻ group, a -COO⁻ group, a halogen atom, a C₁-C₆ alkoxy group; a monohydroxyalkoxy group in which the linear or branched alkyl part is C₁-C₆; a polyhydroxyalkoxy group in which the linear or branched alkyl part is C₁-C₆; an amino group optionally substituted with one or more identical or different, linear or branched C₁-C₆ alkyl radicals, optionally substituted with one or more hydroxyl groups,
n and m, independently of each other, represent an integer between 0 and 4;
An represents a cosmetically acceptable anion or mixture of anions; p being 0 or 1, in order to respect the electrical neutrality of the compound;
and at least one additive chosen from polymers, with the exception of ethyleneoxyalkylphenol-based surfactant polymers.

14. Composition according to the preceding claim, **characterized in that**, in formula (I), the radicals R₁, R₂ and R₃, which may be identical or different, represent a C₁-C₆ alkyl radical; a phenyl radical; an arylalkyl radical in which the aryl part is C₆ and the alkyl part is C₁-C₆; the alkyl, phenyl and arylalkyl radicals being optionally substituted with one or more groups chosen from hydroxyl, halogen, C₁-C₆ alkoxy, amino, (di)alkylamino in which the alkyl part is C₁-C₆, and (di)hydroxyalkylamino in which the alkyl part is C₁-C₆.

15. Composition according to the preceding claim, **characterized in that**, in formula (I), the radicals R₁, R₂ and R₃, which may be identical or different, represent methyl, ethyl, methoxy, hydroxyethyl, 3-sulfopropyl or phenyl radicals.

16. Composition according to either of Claims 14 and 15, **characterized in that**, in formula (I), R₄ and R₅, which may be identical or different, represent a hydrogen atom; a chlorine atom; an amino radical; a (di)alkylamino radical in which the alkyl part is C₁-C₆; a (di)hydroxyalkylamino radical in which the alkyl part is C₁-C₆; a hydroxyl radical; a linear or branched C₁-C₆ alkyl radical or a linear or branched C₁-C₆ alkoxy radical, the said alkyl or alkoxy radicals being optionally substituted with one or more groups chosen from hydroxyl, halogen, C₁-C₆ alkoxy and amino radicals, (di)alkylamino radicals in which the alkyl part is C₁-C₆, and (di)hydroxyalkylamino radicals in which the alkyl part is C₁-C₆.

17. Composition according to the preceding claim, **characterized in that**, in formula (I), R₄ and R₅, which may be identical or different, represent a hydrogen atom or a radical chosen from methyl, ethyl, methoxy, ethoxy, hydroxyl, amino, dimethylamino and dihydroxyethylamino radicals and a chlorine atom.

18. Composition according to any one of Claims 13 to 17, **characterized in that** the content of compound of formula (I) represents 0.001% to 20% by weight of the dye composition and preferably from 0.01% to 10% by weight relative to the weight of the dye composition.

19. Composition according to any one of Claims 13 to 18, **characterized in that** the composition comprises an additive chosen from surfactants of nonionic, anionic, cationic or amphoteric nature.

20. Composition according to any one of Claims 13 to 19, **characterized in that** the polymer is of nonionic, cationic, anionic or amphoteric nature.

21. Composition according to the preceding claim, **characterized in that** the content of surfactants more particularly represents from 0.001% to 30% by weight and preferably from 0.05% to 30% by weight relative to the weight of the dye composition.

22. Composition according to any one of Claims 13 to 21, **characterized in that** the polymer is an associative or non-associative thickening polymer.

23. Composition according to the preceding claim, **characterized in that** the content of associative or non-associative thickening polymer(s) ranges from 0.01% to 10% by weight and more particularly from 0.1% to 5% by weight relative to the weight of the dye composition.

24. Composition according to any one of Claims 13 to 23, **characterized in that** the polymer is a conditioning polymer or a fixing polymer.

25. Composition according to the preceding claim, **characterized in that** the content of conditioning polymer or of fixing polymer ranges from 0.01% to 20% by weight relative to the weight of the dye composition and preferably from 0.1% to 5% by weight relative to the weight of the dye composition.

26. Compound of formula (I') below: 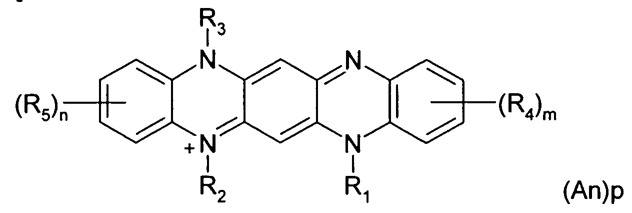 in which formula:
R₁, R₂ and R₃, which may be identical or different, represent a linear or branched C₁-C₂₄ alkyl radical; a C₆-C₃₀ aryl radical; an arylalkyl radical in which the aryl part is C₆-C₃₀ and the linear or branched alkyl part is C₁-C₂₄; on condition that the radicals R₁ and R₃, which may be identical or different, represent alkyl radicals bearing at least one hydroxyl group;
R₄ and R₅, which may be identical or different, represent a hydrogen atom; a halogen atom; a hydroxyl radical; a linear or branched C₁-C₂₄ alkyl radical; a linear or branched C₁-C₂₄ alkoxy radical; a monohydroxyalkoxy group in which the linear or branched alkyl part is C₁-C₆; a polyhydroxyalkoxy group in which the linear or branched alkyl part is C₁-C₆; an amino group optionally substituted with one or more identical or different, linear or branched C₁-C₆ alkyl radicals, optionally substituted with one or more hydroxyl groups; a thiol group; an alkylthio group in which the linear or branched alkyl part is C₁-C₆; a carboxylic group in acid or salified form (with an alkali metal or a substituted or unsubstituted ammonium); an alkoxycarbonyl group in which the linear or branched alkyl part is C₁-C₆; an alkylamide group in which the linear or branched alkyl part is C₁-C₆; or an alkylcarbamyl group in which the linear or branched alkyl part is C₁-C₆; a cyano group; a nitro group; a sulfonyl group; an alkylsulfonyl group in which the linear or branched alkyl part is C₁-C₆; an alkylsulfonylamido group in which the linear or branched alkyl part is C₁-C₆; a C₆-C₃₀ aryl radical optionally substituted with one or more C₁-C₆ alkyl radicals; an arylalkyl radical in which the aryl part is C₆-C₃₀, optionally substituted with one or more C₁-C₆ alkyl radicals and the linear or branched alkyl part is C₁-C₂₄,
the alkyl or aryl radicals per se or the alkyl parts of the compound radicals being optionally substituted with one or more groups chosen from a hydroxyl group, a -SO₃⁻ group, a -COO⁻ group, a halogen atom, a C₁-C₆ alkoxy group; a monohydroxyalkoxy group in which the linear or branched alkyl part is C₁-C₆; a polyhydroxyalkoxy group in which the linear or branched alkyl part is C₁-C₆; an amino group optionally substituted with one or more identical or different, linear or branched C₁-C₆ alkyl radicals, optionally substituted with one or more hydroxyl groups,
n and m, independently of each other, represent an integer between 0 and 4;
An represents a cosmetically acceptable anion or mixture of anions; p being 0 or 1, in order to respect the electrical neutrality of the compound,
with the exception of the following compound: 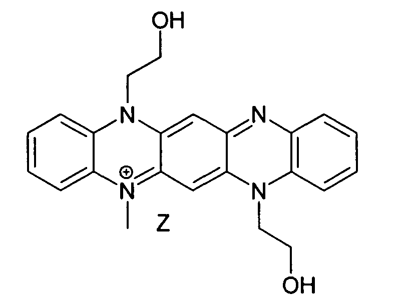
with Z representing an anion.

27. Compound according to Claim 26, corresponding to one of the formulae (i), (ii) and (iii) below:

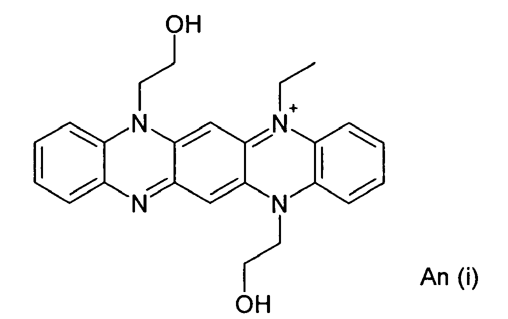

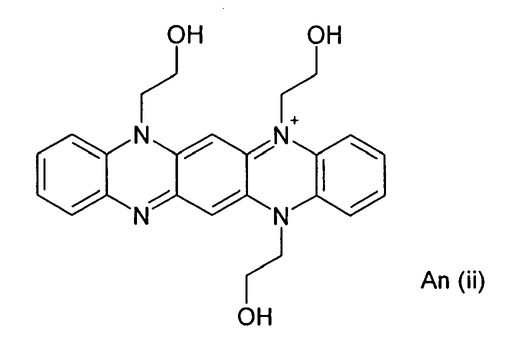

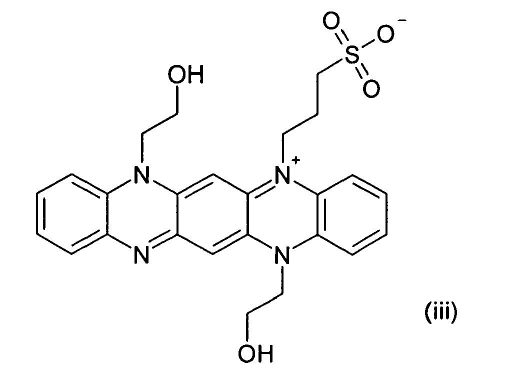

## Patentansprüche

1. Verwendung mindestens einer Verbindung der folgenden Formel (I) als Direktfarbstoff zum Färben von Keratinfasern und insbesondere menschlichen Keratinfasern in einer Farbmittelzusammensetzung, die ein zum Färben geeignetes Medium enthält: 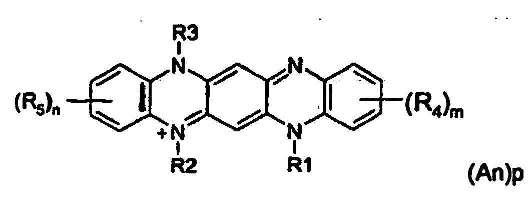 wobei in der Formel bedeuten:
die Gruppen R₁, R₂ und R₃, die identisch oder voneinander verschieden sind, eine geradkettige oder verzweigte C₁₋₂₄-Alkylgruppe; eine C₆₋₃₀-Arylgruppe; eine Arylalkylgruppe, bei der der Arylteil 6 bis 30 Kohlenstoffatome aufweist und der geradkettige oder verzweigte Alkylteil 1 bis 24 Kohlenstoffatome besitzt;
die Gruppen R₄ und R₅, die gleich oder verschieden sind, ein Wasserstoffatom; ein Halogenatom; eine Hydroxygruppe; eine geradkettige oder verzweigte C₁₋₂₄-Alkylgruppe; eine geradkettige oder verzweigte C₁₋₂₄-Alkoxygruppe; eine Monohydroxyalkoxygruppe, bei der der geradkettige oder verzweigte Alkylteil 1 bis 6 Kohlenstoffatome aufweist; eine Polyhydroxyalkoxygruppe, bei der der geradkettige oder verzweigte Alkylteil 1 bis 6 Kohlenstoffatome aufweist; eine Aminogruppe, die unsubstituiert vorliegt oder mit einer oder mehreren C₁₋₆-Alkylgruppen substituiert ist, die gleich oder verschieden sind, geradkettig oder verzweigt vorliegen und unsubstituiert oder mit einer oder mehreren Hydroxygruppen substituiert sind; eine Thiolgruppe; eine Alkylthiogruppe, bei der geradkettige oder verzweigte Alkylteil 1 bis 6 Kohlenstoffatome aufweist; eine Carboxygruppe in Form der Säure oder als Salz (mit einem Alkalimetall oder einer substituierten oder unsubstituierten Ammoniumgruppe); eine Alkoxycarbonylgruppe, bei der der geradkettige oder verzweigte Alkylteil 1 bis 6 Kohlenstoffatome aufweist; eine Alkylamidgruppe, bei der der geradkettige oder verzweigte Alkylteil 1 bis 6 Kohlenstoffatome aufweist; eine Alkylcarbamoylgruppe, bei der der geradkettige oder verzweigte Alkylteil 1 bis 6 Kohlenstoffatome aufweist; eine Cyanogruppe; eine Nitrogruppe; eine Sulfonylgruppe; eine Alkylsulfonylgruppe, deren geradkettiger oder verzweigter Alkylteil 1 bis 6 Kohlenstoffatome aufweist; eine Alkylsulfonylamidogruppe, deren geradkettiger oder verzweigter Alkylteil 1 bis 6 Kohlenstoffatome aufweist; eine C₆₋₃₀-Arylgruppe, die gegebenenfalls mit einer oder mehreren C₁₋₆-Alkylgruppen substituiert ist; eine Arylalkylgruppe, deren Arylteil 6 bis 30 Kohlenstoffatome aufweist und gegebenenfalls mit einer oder mehreren C₁₋₆-Alkylgruppen substituiert ist und deren geradkettiger oder verzweigter Alkylteil 1 bis 24 Kohlenstoffatome aufweist,
wobei die Alkyl- oder Arylgruppen als solche oder die Alkylteile der zusammengesetzten Gruppen gegebenenfalls mit einer oder mehreren Gruppen substituiert sind, die ausgewählt sind unter:
Hydroxy, -SO₃⁻, -COO⁻, Halogen, C₁₋₆-Alkoxy; Monohydroxyalkoxy mit einem geradkettigen oder verzweigten C₁₋₆-Alkylteil; Polyhydroxyalkoxy mit einem geradkettigen oder verzweigten C₁₋₆-Alkylteil; einer Aminogruppe, die unsubstituiert vorliegt oder mit einer oder mehreren C₁₋₆-Alkylgruppen substituiert ist, die gleich oder verschieden sind, geradkettig oder verzweigt vorliegen und unsubstituiert oder mit einer oder mehreren Hydroxygruppen substituiert sind;
n und m unabhängig voneinander 0 oder eine ganze Zahl von 1 bis 4;
An ein kosmetisch akzeptables Anion oder ein Gemisch von kosmetisch akzeptablen Anionen; p 0 oder 1, so dass die Verbindung ungeladen ist.

2. Verwendung nach dem vorhergehenden Anspruch, **dadurch gekennzeichnet, dass** in der Formel (I) die Gruppen R₁, R₂, R₃, die gleich oder verschieden sind, eine C₁₋₆-Alkylgruppe; eine Phenylgruppe; eine Arylalkylgruppe, deren Arylteil 6 Kohlenstoffatome und deren Alkylteil 1 bis 6 Kohlenstoffatome aufweist, bedeuten; wobei die Alkyl-, Phenyl-, Arylalkylgruppen gegebenenfalls mit einer oder mehreren Gruppen substituiert sind, die unter den Gruppen Hydroxy, -SO₃⁻, -COO⁻, Halogen, C₁₋₆-Alkoxy, Amino, (Di)alkylamino mit einem C₁₋₆-Alkylteil, (Di)hydroxyalkylamino mit einem C₁₋₆-Alkylteil ausgewählt sind.

3. Verwendung nach dem vorhergehenden Anspruch, **dadurch gekennzeichnet, dass** in der Formel (I) die Gruppen R₁, R₂, R₃, die gleich oder verschieden sind, Methyl, Ethyl, Methoxy, Hydroxyethyl, 3-Sulfopropyl, Phenyl bedeuten.

4. Verwendung nach einem der vorhergehenden Ansprüche, **dadurch gekennzeichnet, dass** in der Formel (I) die Gruppen R₄ und R₅, die gleich oder verschieden sind, ein Wasserstoffatom; ein Chloratom; eine Aminogruppe; eine (Di)alkylaminogruppe, bei der der Alkylteil 1 bis 6 Kohlenstoffatome aufweist; eine (Di)hydroxyalkylaminogruppe, deren Alkylteil 1 bis 6 Kohlenstoffatome aufweist; eine Hydroxygruppe; eine geradkettige oder verzweigte C₁₋₆-Alkylgruppe, eine geradkettige oder verzweigte C₁₋₆-Alkoxygruppe bedeuten, wobei die Alkyl- oder Alkoxygruppen gegebenenfalls mit einer oder mehreren Gruppen substituiert sind, die unter den Gruppen Hydroxy, Halogen, C₁₋₆-Alkoxy, Amino, (Di)alkylamino mit einem C₁₋₆-Alkylteil, (Di)hydroxyalkylamino mit einem C₁₋₆-Alkylteil ausgewählt sind.

5. Verwendung nach dem vorhergehenden Anspruch, **dadurch gekennzeichnet, dass** in der Formel (I) die Gruppen R₄ und R₅, die gleich oder verschieden sind, ein Wasserstoffatom, eine Gruppe, die unter den Gruppen Methyl, Ethyl, Methoxy, Ethoxy, Hydroxy, Amino, Dimethylamino, Dihydroxyethylamino ausgewählt ist, oder ein Chloratom bedeuten.

6. Verwendung nach einem der vorhergehenden Ansprüche, **dadurch gekennzeichnet, dass** der Mengenanteil der Verbindung der Formel (I) 0,001 bis 20 Gew.-% der Farbmittelzusammensetzung und vorzugsweise 0,01 bis 10 Gew.-%, bezogen auf das Gesamtgewicht der Farbmittelzusammensetzung, ausmacht.

7. Verwendung nach einem der vorhergehenden Ansprüche, **dadurch gekennzeichnet, dass** die Zusammensetzung mindestens einen von der Verbindung der Formel (I) verschiedenen, ergänzenden Direktfarbstoff enthält, der unter den nichtionischen oder ionischen und vorzugsweise nichtionischen oder kationischen Verbindungen ausgewählt ist.

8. Verwendung nach dem vorhergehenden Anspruch, **dadurch gekennzeichnet, dass** der ergänzende Direktfarbstoff unter den Acridin-Farbstoffen, Acridon-Farbstoffen, Anthranthron-Farbstoffen, Anthrapyrimidin-Farbstoffen, Anthrachinon-Farbstoffen, Azin-Farbstoffen, Azofarbstoffen, Azomethin-Farbstoffen, Benzanthron-Farbstoffen, Benzimidazol-Farbstoffen, Benzimidazolon-Farbstoffen, Benzindol-Farbstoffen, Benzoxazol-Farbstoffen, Benzopyran-Farbstoffen, Benzothiazol-Farbstoffen, Benzochinon-Farbstoffen, Bisazin-Farbstoffen, Bisisoindolin-Farbstoffen, Carboxanilid-Farbstoffen, Cumarin-Farbstoffen, Cyanin-Farbstoffen, Diazin-Farbstoffen, Diketopyrrolopyrrol-Farbstoffen, Dioxazin-Farbstoffen, Diphenylamin-Farbstoffen, Diphenylmethan-Farbstoffen, Dithiazin-Farbstoffen, Flavanthron-Farbstoffen, Flavon-Farbstoffen, Fluorindin-Farbstoffen, Formazan-Farbstoffen, Hydrazon-Farbstoffen, Hydroxyketon-Farbstoffen, Indamin-Farbstoffen, Indanthron-Farbstoffen, Indigoid-Farbstoffen, Indophenol-Farbstoffen, Indoanilin-Farbstoffen, Isoindolin-Farbstoffen, Isoindolinon-Farbstoffen, Isoviolanthron-Farbstoffen, Lacton-Farbstoffen, Methin-Farbstoffen, Naphthalimid-Farbstoffen, Naphthanilid-Farbstoffen, Naphtholactam-Farbstoffen, Naphthochinon-Farbstoffen, Nitro-Farbstoffen, Oxadiazol-Farbstoffen, Oxazin-Farbstoffen, Perilon-Farbstoffen, Perinon-Farbstoffen, Perylen-Farbstoffen, Phenazin-Farbstoffen, Phenothiazin-Farbstoffen, Phthalocyanin-Farbstoffen, Polyen/Carotinoid-Farbstoffen, Porphyrin-Farbstoffen, Pyranthron-Farbstoffen, Pyrazolanthron-Farbstoffen, Pyrazolon-Farbstoffen, Pyrimidinoanthron-Farbstoffen, Pyronin-Farbstoffen, Chinacridon-Farbstoffen, Chinolin-Farbstoffen, Chinophthalon-Farbstoffen, Squaran-Farbstoffen, Stilben-Farbstoffen, Tetrazolium-Farbstoffen, Thiazin-Farbstoffen, Thioindigo, Thiopyronin-Farbstoffen, Triarylmethan-Farbstoffen, Xanthen-Farbstoffen oder deren Gemischen ausgewählt ist.

9. Verwendung nach dem vorhergehenden Anspruch, **dadurch gekennzeichnet, dass** der Mengenanteil des oder der von den Verbindungen der Formel (I) verschiedenen, ergänzenden Direktfarbstoffe(s) 0,0005 bis 12 Gew.-%, bezogen auf das Gewicht der Farbmittelzusammensetzung, und vorzugsweise 0,005 bis 6 Gew.-%, bezogen auf das Gewicht der Farbmittelzusammensetzung, ausmacht.

10. Verwendung nach einem der vorhergehenden Ansprüche, **dadurch gekennzeichnet, dass** die Farbmittelzusammensetzung mindestens ein Oxidationsmittel enthält.

11. Verwendung nach dem vorhergehenden Anspruch, **dadurch gekennzeichnet, dass** das Oxidationsmittel unter Wasserstoffperoxid, Harnstoffperoxid, Alkalimetallbromaten, Alkalimetallferricyaniden, Salzen von Persäuren, wie Perboraten und Persulfaten, Enzymen oder deren Gemischen ausgewählt ist.

12. Verfahren zum Färben von Keratinfasern und insbesondere menschlichen Keratinfasern, bei dem die folgenden Schritte durchgeführt werden:
a) die trockenen oder feuchten Fasern werden mit einer Zusammensetzung, die mindestens eine Verbindung der Formel (I), wie sie in einem der vorhergehenden Ansprüche beschrieben wurde, enthält, während einer für die Bildung der Färbung ausreichenden Zeitspanne in Kontakt gebracht,
b) die Fasern werden gegebenenfalls gespült,
c) die Fasern werden gegebenenfalls gewaschen und gespült,
d) die Fasern werden getrocknet oder trocknen gelassen.

13. Farbmittelzusammensetzung, die in einem zum Färben von Keratinfasern und insbesondere menschlichen Keratinfasern geeigneten Medium als Direktfarbstoff mindestens eine Verbindung der folgenden Formel (I): 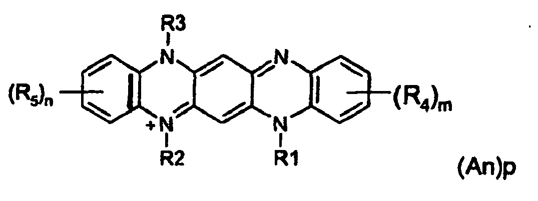 wobei in der Formel bedeuten:
die Gruppen R₁, R₂ und R₃, die identisch oder voneinander verschieden sind, eine geradkettige oder verzweigte C₁₋₂₄-Alkylgruppe; eine C₆₋₃₀-Arylgruppe; eine Arylalkylgruppe, bei der der Arylteil 6 bis 30 Kohlenstoffatome aufweist und der geradkettige oder verzweigte Alkylteil 1 bis 24 Kohlenstoffatome besitzt;
die Gruppen R₄ und R₅, die gleich oder verschieden sind, ein Wasserstoffatom; ein Halogenatom; eine Hydroxygruppe; eine geradkettige oder verzweigte C₁₋₂₄-Alkylgruppe; eine geradkettige oder verzweigte C₁₋₂₄-Alkoxygruppe; eine Monohydroxyalkoxygruppe, bei der der geradkettige oder verzweigte Alkylteil 1 bis 6 Kohlenstoffatome aufweist; eine Polyhydroxyalkoxygruppe, bei der der geradkettige oder verzweigte Alkylteil 1 bis 6 Kohlenstoffatome aufweist; eine Aminogruppe, die unsubstituiert vorliegt oder mit einer oder mehreren C₁₋₆-Alkylgruppen substituiert ist, die gleich oder verschieden sind, geradkettig oder verzweigt vorliegen und unsubstituiert oder mit einer oder mehreren Hydroxygruppen substituiert sind; eine Thiolgruppe; eine Alkylthiogruppe, bei der geradkettige oder verzweigte Alkylteil 1 bis 6 Kohlenstoffatome aufweist; eine Carboxygruppe in Form der Säure oder als Salz (mit einem Alkalimetall oder einer substituierten oder unsubstituierten Ammoniumgruppe); eine Alkoxycarbonylgruppe, bei der der geradkettige oder verzweigte Alkylteil 1 bis 6 Kohlenstoffatome aufweist; eine Alkylamidgruppe, bei der der geradkettige oder verzweigte Alkylteil 1 bis 6 Kohlenstoffatome aufweist; eine Alkylcarbamoylgruppe, bei der der geradkettige oder verzweigte Alkylteil 1 bis 6 Kohlenstoffatome aufweist; eine Cyanogruppe; eine Nitrogruppe; eine Sulfonylgruppe; eine Alkylsulfonylgruppe, deren geradkettiger oder verzweigter Alkylteil 1 bis 6 Kohlenstoffatome aufweist; eine Alkylsulfonylamidogruppe, deren geradkettiger oder verzweigter Alkylteil 1 bis 6 Kohlenstoffatome aufweist; eine C₆₋₃₀-Arylgruppe, die gegebenenfalls mit einer oder mehreren C₁₋₆-Alkylgruppen substituiert ist; eine Arylalkylgruppe, deren Arylteil 6 bis 30 Kohlenstoffatome aufweist und gegebenenfalls mit einer oder mehreren C₁₋₆-Alkylgruppen substituiert ist und deren geradkettiger oder verzweigter Alkylteil 1 bis 24 Kohlenstoffatome aufweist,
wobei die Alkyl- oder Arylgruppen als solche oder die Alkylteile der zusammengesetzten Gruppen gegebenenfalls mit einer oder mehreren Gruppen substituiert sind, die ausgewählt sind unter:
Hydroxy, -SO₃⁻, -COO⁻, Halogen, C₁₋₆-Alkoxy; Monohydroxyalkoxy mit einem geradkettigen oder verzweigten C₁₋₆-Alkylteil; Polyhydroxyalkoxy mit einem geradkettigen oder verzweigten C₁₋₆-Alkylteil; einer Aminogruppe, die unsubstituiert vorliegt oder mit einer oder mehreren C₁₋₆-Alkylgruppen substituiert ist, die gleich oder verschieden sind, geradkettig oder verzweigt vorliegen und unsubstituiert oder mit einer oder mehreren Hydroxygruppen substituiert sind;
n und m unabhängig voneinander 0 oder eine ganze Zahl von 1 bis 4;
An ein kosmetisch akzeptables Anion oder ein Gemisch von kosmetisch akzeptablen Anionen; p 0 oder 1, so dass die Verbindung ungeladen ist;
und mindestens einen Zusatzstoff enthält, der unter den Polymeren ausgewählt ist, wobei grenzflächenaktive Polymere ausgenommen sind, die von Ethylenoxyalkylphenol abgeleitet sind.

14. Zusammensetzung nach dem vorhergehenden Anspruch, **dadurch gekennzeichnet, dass** in der Formel (I) die Gruppen R₁, R₂, R₃, die gleich oder verschieden sind, eine C₁₋₆-Alkylgruppe; eine Phenylgruppe; eine Arylalkylgruppe, deren Arylteil 6 Kohlenstoffatome und deren Alkylteil 1 bis 6 Kohlenstoffatome aufweist, bedeuten; wobei die Alkyl-, Phenyl-, Arylalkylgruppen gegebenenfalls mit einer oder mehreren Gruppen substituiert sind, die unter den Gruppen Hydroxy, Halogen, C₁₋₆-Alkoxy, Amino, (Di)alkylamino mit einem C₁₋₆-Alkylteil, (Di)hydroxyalkylamino mit einem C₁₋₆-Alkylteil ausgewählt sind.

15. Verwendung nach dem vorhergehenden Anspruch, **dadurch gekennzeichnet, dass** in der Formel (I) die Gruppen R₁, R₂, R₃, die gleich oder verschieden sind, Methyl, Ethyl, Methoxy, Hydroxyethyl, 3-Sulfopropyl, Phenyl bedeuten.

16. Zusammensetzung nach einem der Ansprüche 14 oder 15, **dadurch gekennzeichnet, dass** in der Formel (I) die Gruppen R₄ und R₅, die gleich oder verschieden sind, ein Wasserstoffatom; ein Chloratom; eine Aminogruppe; eine (Di)alkylaminogruppe, bei der der Alkylteil 1 bis 6 Kohlenstoffatome aufweist; eine (Di)hydroxyalkylaminogruppe, deren Alkylteil 1 bis 6 Kohlenstoffatome aufweist; eine Hydroxygruppe; eine geradkettige oder verzweigte C₁₋₆-Alkylgruppe, eine geradkettige oder verzweigte C₁₋₆-Alkoxygruppe bedeuten, wobei die Alkyl- oder Alkoxygruppen gegebenenfalls mit einer oder mehreren Gruppen substituiert sind, die unter den Gruppen Hydroxy, Halogen, C₁₋₆-Alkoxy, Amino, (Di)alkylamino mit einem C₁₋₆-Alkylteil, (Di)hydroxyalkylamino mit einem C₁₋₆-Alkylteil ausgewählt sind.

17. Zusammensetzung nach dem vorhergehenden Anspruch, **dadurch gekennzeichnet, dass** in der Formel (I) die Gruppen R₄ und R₅, die gleich oder verschieden sind, ein Wasserstoffatom, eine Gruppe, die unter den Gruppen Methyl, Ethyl, Methoxy, Ethoxy, Hydroxy, Amino, Dimethylamino, Dihydroxyethylamino ausgewählt ist, oder ein Chloratom bedeuten.

18. Zusammensetzung nach einem der Ansprüche 13 bis 17, **dadurch gekennzeichnet, dass** der Gehalt der Verbindung der Formel (I) 0,001 bis 20 Gew.-% der Farbmittelzusammensetzung und vorzugsweise 0,01 bis 10 Gew.-%, bezogen auf das Gesamtgewicht der Farbmittelzusammensetzung, ausmacht.

19. Zusammensetzung nach einem der Ansprüche 13 bis 18, **dadurch gekennzeichnet, dass** die Zusammensetzung einen Zusatzstoff enthält, der unter den grenzflächenaktiven Stoffen vom nichtionischen, anionischen, kationischen oder amphoteren Typ ausgewählt ist.

20. Zusammensetzung nach einem der Ansprüche 13 bis 19, **dadurch gekennzeichnet, dass** das Polymer vom nichtionischen, kationischen, anionischen oder amphoteren Typ ist.

21. Zusammensetzung nach dem vorhergehenden Anspruch, **dadurch gekennzeichnet, dass** der Gehalt der grenzflächenaktiven Stoffe insbesondere 0,001 bis 30 Gew.-% und vorzugsweise 0,05 bis 30 Gew.-%, bezogen auf das Gesamtgewicht der Farbmittelzusammensetzung, ausmacht.

22. Zusammensetzung nach einem der Ansprüche 13 bis 21, **dadurch gekennzeichnet, dass** das Polymer ein assoziatives oder nichtassoziatives, verdickendes Polymer ist.

23. Zusammensetzung nach dem vorhergehenden Anspruch, **dadurch gekennzeichnet, dass** der Mengenanteil des oder der assoziativen oder nichtassoziativen, verdickenden Polymere im Bereich von 0,01 bis 10 Gew.-% und insbesondere 0,1 bis 5 Gew.-%, bezogen auf das Gewicht der Farbmittelzusammensetzung, liegt.

24. Zusammensetzung nach einem der Ansprüche 13 bis 23, **dadurch gekennzeichnet, dass** das Polymer ein konditionierendes Polymer oder ein fixierendes Polymer ist.

25. Zusammensetzung nach dem vorhergehenden Anspruch, **dadurch gekennzeichnet, dass** der Mengenanteil des konditionierenden Polymers oder fixierenden Polymers im Bereich von 0,01 bis 20 Gew.-%, bezogen auf das Gewicht der Farbmittelzusammensetzung, und vorzugsweise 0,1 bis 5 Gew.-%, bezogen auf das Gewicht der Farbmittelzusammensetzung, liegt.

26. Verbindung der folgenden Formel (I'): 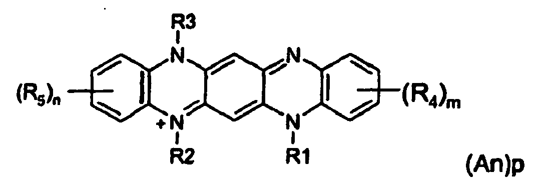 wobei in der Formel bedeuten:
die Gruppen R₁, R₂ und R₃, die identisch oder voneinander verschieden sind, eine geradkettige oder verzweigte C₁₋₂₄-Alkylgruppe;
eine C₆₋₃₀-Arylgruppe; eine Arylalkylgruppe, bei der der Arylteil 6 bis 30 Kohlenstoffatome aufweist und der geradkettige oder verzweigte Alkylteil 1 bis 24 Kohlenstoffatome besitzt, mit der Maßgabe, dass die Gruppen R₁ und R₃, die identisch oder voneinander verschieden sind, Alkylgruppen bedeuten, die mindestens eine Hydroxygruppe tragen;
die Gruppen R₄ und R₅, die gleich oder verschieden sind, ein Wasserstoffatom; ein Halogenatom; eine Hydroxygruppe; eine geradkettige oder verzweigte C₁₋₂₄-Alkylgruppe; eine geradkettige oder verzweigte C₁₋₂₄-Alkoxygruppe; eine Monohydroxyalkoxygruppe, bei der der geradkettige oder verzweigte Alkylteil 1 bis 6 Kohlenstoffatome aufweist; eine Polyhydroxyalkoxygruppe, bei der der geradkettige oder verzweigte Alkylteil 1 bis 6 Kohlenstoffatome aufweist; eine Aminogruppe, die unsubstituiert vorliegt oder mit einer oder mehreren C₁₋₆-Alkylgruppen substituiert ist, die gleich oder verschieden sind, geradkettig oder verzweigt vorliegen und unsubstituiert oder mit einer oder mehreren Hydroxygruppen substituiert sind; eine Thiolgruppe; eine Alkylthiogruppe, bei der geradkettige oder verzweigte Alkylteil 1 bis 6 Kohlenstoffatome aufweist; eine Carboxygruppe in Form der Säure oder als Salz (mit einem Alkalimetall oder einer substituierten oder unsubstituierten Ammoniumgruppe); eine Alkoxycarbonylgruppe, bei der der geradkettige oder verzweigte Alkylteil 1 bis 6 Kohlenstoffatome aufweist; eine Alkylamidgruppe, bei der der geradkettige oder verzweigte Alkylteil 1 bis 6 Kohlenstoffatome aufweist; eine Alkylcarbamoylgruppe, bei der der geradkettige oder verzweigte Alkylteil 1 bis 6 Kohlenstoffatome aufweist; eine Cyanogruppe; eine Nitrogruppe; eine Sulfonylgruppe; eine Alkylsulfonylgruppe, deren geradkettiger oder verzweigter Alkylteil 1 bis 6 Kohlenstoffatome aufweist; eine Alkylsulfonylamidogruppe, deren geradkettiger oder verzweigter Alkylteil 1 bis 6 Kohlenstoffatome aufweist; eine C₆₋₃₀-Arylgruppe, die gegebenenfalls mit einer oder mehreren C₁₋₆-Alkylgruppen substituiert ist; eine Arylalkylgruppe, deren Arylteil 6 bis 30 Kohlenstoffatome aufweist und gegebenenfalls mit einer oder mehreren C₁₋₆-Alkylgruppen substituiert ist und deren geradkettiger oder verzweigter Alkylteil 1 bis 24 Kohlenstoffatome aufweist,
wobei die Alkyl- oder Arylgruppen als solche oder die Alkylteile der zusammengesetzten Gruppen gegebenenfalls mit einer oder mehreren Gruppen substituiert sind, die ausgewählt sind unter:
Hydroxy, -SO₃⁻, -COO⁻, Halogen, C₁₋₆-Alkoxy; Monohydroxyalkoxy mit einem geradkettigen oder verzweigten C₁₋₆-Alkylteil; Polyhydroxyalkoxy mit einem geradkettigen oder verzweigten C₁₋₆-Alkylteil; einer Aminogruppe, die unsubstituiert vorliegt oder mit einer oder mehreren C₁₋₆-Alkylgruppen substituiert ist, die gleich oder verschieden sind, geradkettig oder verzweigt vorliegen und unsubstituiert oder mit einer oder mehreren Hydroxygruppen substituiert sind;
n und m unabhängig voneinander 0 oder eine ganze Zahl von 1 bis 4;
An ein kosmetisch akzeptables Anion oder ein Gemisch von kosmetisch akzeptablen Anionen; p 0 oder 1, so dass die Verbindung ungeladen ist, wobei die folgende Verbindung ausgenommen ist: 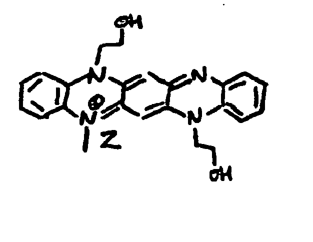
wobei Z ein Anion bedeutet.

27. Verbindung nach Anspruch 26, die einer der folgenden Formeln (i), (ii), (iii) entspricht:
